# EUROPÄISCHE PATENTSCHRIFT

(11) **EP 3 623 798 B1**
(45) Veröffentlichungstag und Bekanntmachung des Hinweises auf die Patenterteilung: **03.11.2021**
(21) Anmeldenummer: 18194332.5
(22) Anmeldetag: 13.09.2018
(51) Int. Cl.: G01N 21/64, G02B 21/36, G06F 3/00, G02B 21/16

(54) **VERFAHREN UND VORRICHTUNG ZUM ERFASSEN UND DARSTELLEN EINES IMMUNFLUORESZENZBILDES EINER BIOLOGISCHEN PROBE**
METHOD AND DEVICE FOR DETECTING AND REPRESENTING AN IMMUNOFLUORESCENCE IMAGE OF A BIOLOGICAL SAMPLE
PROCÉDÉ ET DISPOSITIF DE DÉTECTION ET DE REPRÉSENTATION D'UNE IMAGE D'IMMUNOFLUORESCENCE D'UN ÉCHANTILLON BIOLOGIQUE

(43) Veröffentlichungstag der Anmeldung: 18.03.2020
(73) Patentinhaber: Euroimmun Medizinische Labordiagnostika AG, 23560 Lübeck (DE)
(72) Erfinder: SUMPF, Tilman Johannes, 23552 Lübeck (DE); PAPE, Michael, 23552 Lübeck (DE); HAGEN-EGGERT, Martin, 23568 Lübeck (DE); PIEPKE, Christian, 23568 Lübeck (DE); LAUDAN, Thomas, 23566 Lübeck (DE); FALKERT, Michael, 23858 Reinfeld (DE); MORRIN, Markus, 23564 Lübeck (DE); RENTZSCH, Kristin, 23611 Bad Schwartau (DE)

(56) Entgegenhaltungen:
- EP-A1- 2 000 842
- EP-A1- 2 557 446
- EP-A1- 2 690 482
- JP-A- H08 223 563

## Beschreibung

Aus dem Stand der Technik sind Verfahren und Vorrichtungen bekannt, um sogenannte Immunfluoreszenzbilder von biologischen Proben mittels Bildsensoren zu erfassen und auf Anzeigeeinheiten anzuzeigen.

Eine solche biologische Probe ist beispielsweise eine Gewebeprobe, welche einem Menschen oder einem Tier entnommen wurde. Im Zuge einer sogenannten direkten Immunfluoreszenz können beispielsweise Zellbestandteile innerhalb des Gewebes sichtbar gemacht werden, indem das Gewebe mit Antikörpern eines bestimmten Typs inkubiert wird, wobei die Antikörper spezifisch mit bestimmten Antigenen des Gewebes reagieren. Hierbei sind an die Antikörper sogenannte Fluoreszenzfarbstoffe gebunden, sodass die zu detektierenden Strukturen dann in einem Immunfluoreszenzbild sichtbar gemacht werden können. Hierzu wird der Fluoreszenzfarbstoff mit einem Anregungslicht einer bestimmten Anregungswellenlänge bestrahlt, sodass dann die Fluoreszenzstrahlung, welche eine andere Wellenlänge als die Anregungswellenlänge aufweist, von dem Gewebe emittiert wird und mittels eines Objektives hin zu einem Bildsensor geführt werden kann um dann von dem Bildsensor erfasst zu werden.

Im Zuge einer sogenannten indirekten Immunfluoreszenz wird beispielsweise als die biologische Probe ein Gewebe, beispielsweise Affenleber, verwendet und mit einem Blutserum eines Patienten in einem ersten Inkubationsschritt inkubiert, um eine mögliche Bindung von Antikörpern in dem Serum des Patienten an bestimmte Antigene des Gewebes zu detektieren. Es wird ferner auch eine zweite Inkubation mit Antikörpern einer zweiten Art durchgeführt, welche dann an die ersten Antikörper aus dem Blutserum anbindet, wobei der zweite Antikörper eben wiederum mit einem Fluoreszenzfarbstoff markiert ist. Auch bei der indirekten Immunfluoreszenz wird dann das inkubierte Gewebe mit dem Anregungslicht der Anregungswellenlänge bestrahlt, sodass eine Bindung von Antikörpern des Patienten an bestimmte Antigene in dem Gewebe durch eine Fluoreszenzstrahlung des Fluoreszenzfarbstoffs mit einer Fluoreszenzwellenlänge, welche sich von der Anregungslichtwellenlänge unterscheidet, sichtbar wird.

Das Dokument EP2000842A1 offenbart ein Verfahren zum Erfassen und Darstellen eines Mikroskopbildes, bei welchem eine Probe mit Anregungsstrahlung beleuchtet wird und bei welchem in der Relativposition zwischen Probe und optischem System mittels eines Nutzers verwendet werden kann, sodass nach Beendigung der Nutzereingabe ein sogenanntes "still image" angezeigt wird.

Die biologische Probe kann auch durch sogenannte Antigen-Spots gegeben sein, welche nach Inkubation der Antigen-Spots mit einem Blutserum eines Patienten an die Antigen-Spots gebundene Antikörper des Patienten aufweisen. An die Antikörper des Patienten können dann wiederum nach Durchführen eines weiteren Inkubationsschrittes sogenannte zweite Antikörper gebunden sein, welche ihrerseits mit einem Fluoreszenzfarbstoff markiert sind.

Derartig gewonnene Immunfluoreszenzbilder können dann Gegenstand einer klinischen Befundung sein.

Bekannt sind insbesondere Vorrichtungen wie beispielsweise Mikroskope, bei welchen ein Nutzer manuell eine Ausrichtung der Probe gegenüber dem Mikroskop bzw. dem Objektiv des Mikroskops vornehmen kann, um in dem gewonnenen Immunfluoreszenzbild einen bestimmten Bereich bzw. einen bestimmten Ausschnitt der Probe sichtbar zu machen.

Aufgabe der vorliegenden Erfindung ist es, eine Vorrichtung und ein Verfahren für einen Nutzer bereitzustellen, bei welcher bzw. bei welchem ein Erfassen von Immunfluoreszenzbildern an unterschiedlichen Ausschnitten bzw. Bereichen einer Probe für einen Nutzer möglichst einfach und gut handhabbar bzw. gut bedienbar und sicher durchführbar gestaltet ist.

Die erfindungsgemäße Aufgabe wird gelöst durch ein Verfahren nach dem Patentanspruch 1 sowie eine Vorrichtung nach dem Patentanspruch 12.

Vorgeschlagen wird ein Verfahren zum Erfassen und Darstellen eines Immunfluoreszenzbildes einer biologischen Probe. In einem ersten Betriebszustand führt das Verfahren bestimmte Verfahrensschritte durch. Während des ersten Betriebszustandes wird die Probe fortdauernd mit der Anregungsstrahlung beleuchtet. Hierdurch wird insbesondere ein fortdauerndes Emittieren der Fluoreszenzstrahlung durch die Probe erzeugt. Eine Relativposition zwischen der Probe und einem optischen System, welches von der Probe emittierte Fluoreszenzstrahlung hin zu wenigstens einem Bildsensor führt, wird in Abhängigkeit einer Bewegungsgeste eines Nutzers verändert. Hierdurch wird insbesondere erreicht, dass der Nutzer durch die Bewegungsgeste bestimmen kann, welcher Ausschnitt der Probe mittels des optischen Systems und des Bildsensors in dem Immunfluoreszenzbild sichtbar wird. Während des ersten Betriebszustandes wird die Fluoreszenzstrahlung mittels mehrerer Sensorpixel wenigstens eines Bildsensors unter Verwendung einer ersten Erfassungszeitdauer erfasst. Auf Basis von resultierenden Sensorpixelwerten der Sensorpixel wird dann ein Digitalbild bestimmt und dieses angezeigt.

In dem ersten Betriebszustand werden das Erfassen der Fluoreszenzstrahlung sowie das Bestimmen und das Anzeigen des Digitalbildes zu aufeinanderfolgenden Zeitpunkten mit einer bestimmten Wiederholungsfrequenz wiederholt. Hierdurch werden also insbesondere in dem Fall, dass eine Bewegungsgeste fortlaufend durchgeführt wird, jeweils unterschiedliche Ausschnitte bzw. Bereiche der Probe in dem Immunfluoreszenzbild erfasst und zu den jeweils korrespondierenden, aufeinanderfolgenden Zeitpunkten in dem Digitalbild angezeigt. Der Nutzer kann also insbesondere durch Durchführung der Bewegungsgeste bestimmen, welcher Ausschnitt der Probe in dem Digitalbild dargestellt bzw. angezeigt wird. Hierbei erfolgt eben insbesondere in dem ersten Betriebszustand das Erfassen der Fluoreszenzstrahlung für ein Digitalbild eines Zeitpunktes mit der ersten Erfassungszeitdauer. Die maximale Wiederholungsfrequenz zur Darstellung der aufeinanderfolgenden Digitalbilder in dem ersten Betriebszustand wird also insbesondere durch diese erste Erfassungszeitdauer bestimmt und ist der Kehrwert der ersten Erfassungszeitdauer.

Bei Detektieren einer Beendigung der Bewegungsgeste wird von dem ersten Betriebszustand in einen weiteren, zweiten Betriebszustand gewechselt. In dem zweiten Betriebszustand wird zunächst die Probe mit der Anregungsstrahlung zur Anregung der Fluoreszenzstrahlung des Fluoreszenzfarbstoffes beleuchtet. Es erfolgt dann ein Erfassen von von der Probe emittierter Fluoreszenzstrahlung mittels der mehreren Sensorpixeln unter Verwendung einer zweiten Erfassungszeitdauer. Diese zweite Erfassungszeitdauer ist größer als die erste Erfassungszeitdauer. Auf Basis resultierender Sensorpixelwerte der Sensorpixel wird dann ein weiteres Digitalbild erzeugt. In dem zweiten Betriebszustand wird das Erfassen der emittierten Fluoreszenzstrahlung und das Beleuchten der Probe nach Ablauf der zweiten Erfassungszeitdauer beendet. Ferner wird nach dem Beenden des Erfassens der Fluoreszenzstrahlung und dem Beenden des Beleuchtens der Probe das weitere Digitalbild fortdauern angezeigt.

Um dem Leser einen Vorteil oder mehrere Vorteile, welche möglicherweise durch die Erfindung erreicht werden können, genauer zu erläutern, folgen nun im Weiteren detaillierte Erläuterungen. Für einen Nutzer ist es beispielsweise für eine tatsächliche Befundung eines Patienten notwendig, nicht nur einen Bildausschnitt einer inkubierten Probe in einem Immunfluoreszenzbild zu betrachten, sondern verschiedene Bereiche eines Gewebes in einer jeweils hohen Vergrößerungsstufe betrachten zu können. Hierzu muss der Nutzer eine Relativposition des optischen Systems, welches die emittierte Fluoreszenzstrahlung von Gewebe hin zu dem Bildsensor führt, gegenüber der Probe verändern können. Insbesondere bei einer hohen optischen Vergrößerung möchte der Nutzer solche unterschiedlichen Bereiche dann ggf. in hoher Bildqualität betrachten können, wenn die Relativposition zwischen der Probe und dem optischen System unverändert bleibt, so dass während des zweiten Betriebszustandes konstant der gleiche Bildausschnitt der Probe in Form des weiteren Digitalbildes dargestellt wird. Der Nutzer möchte das dargestellte weitere Digitalbild ggf. eine längere Zeit lang betrachten, um Strukturen oder Färbungen detailliert in Augenschein zu nehmen und für sich zu entscheiden, ob er ggf. durch Eingabe einer weiteren Bewegungsgeste einen anderen Bildausschnitt betrachten möchte. Dadurch, dass in dem zweiten Betriebszustand nach dem Ablauf der zweiten Erfassungszeitdauer das weitere Digitalbild fortdauernd angezeigt wird und dass ferner das Beleuchten der Probe durch die Anregungsstrahlung beendet wird, wird ein sogenanntes Ausbrennen der biologischen Probe aufgrund eines Beleuchtens mit der Anregungsstrahlung minimiert. Würde die Probe während des zweiten Betriebszustandes fortdauernd mit der Anregungsstrahlung beleuchtet werden, so würde die Probe deutlich früher ausbrennen und nicht mehr für eine Mikroskopie verwendbar sein, welches als ein technisches Problem anzusehen ist. Für den Nutzer ergibt sich durch das hier vorgeschlagene Verfahren der Eindruck, dass er nicht nur während des Durchführens seiner Bewegungsgeste fortdauernd erfasste Bilder als Repräsentationen eines realen Mikroskopbildes angezeigt bekommt, sondern dass ihm auch während des zweiten Betriebszustandes weiterhin ein fortdauerndes Mikroskopbild angezeigt wird, da er das weitere Digitalbild fortdauernd angezeigt bekommt. Durch die hier vorgeschlagene Lösung wird also erreicht, dass der Nutzer den Eindruck einer fortdauernden Mikroskopie bzw. einer sogenannten "Live-Mikroskopie" auf der Anzeigeeinheit erhält, obwohl während des zweiten Betriebszustandes nach Ablauf der zweiten Erfassungszeitdauer und nach dem Bestimmen des weiteren Digitalbildes keine weitere Erfassung von Fluoreszenzstrahlung bei gleichzeitiger Beleuchtung des Gewebes mit der Anregungsstrahlung erfolgt. Der Nutzer bemerkt also gar nicht, dass während zumindest eines zeitlichen Teilabschnittes des zweiten Betriebszustandes keinerlei Bilderfassung mehr erfolgt und dass die biologische Probe dennoch gleichzeitig hinsichtlich eines Ausbrennens geschont wird.

Ein weiteres technisches Problem besteht darin, dass der Nutzer zum Einen während der Durchführung einer Bewegungsgeste, beispielsweise durch Streichen eines Fingers auf einer berührungsempfindlichen Anzeigeoberfläche wie einem Touch-Screen, ein möglichst fließendes Bild bzw. ruckelfreies Bild in der Darstellung auf der Anzeigeeinheit erwartet, um das Gefühl zu haben, dass der Bildausschnitt bzw. die Positioniereinheit seiner Bewegungsgeste ohne Verzögerung folgt und er nicht durch ein verzögertes Anzeigen von zu aufeinanderfolgenden Zeitpunkten gewonnener Digitalbilder den Eindruck hat, dass die Bilderfassungsvorrichtung verzögert den Bildausschnitt wählt und die Digitalbilder darstellt. Gleichzeitig ist es aber für den Nutzer notwendig, dass nach Durchführung seiner Bewegungsgeste und der damit einhergehenden Auswahl eines bestimmten Bildausschnittes der Probe dann in dem zweiten Betriebszustand das weitere Digitalbild in hinreichender bzw. relativ hoher Qualität angezeigt bekommt, da bei Immunfluoreszenzbildern kleinste Bilddetails für die Befundung durch den Nutzer von Interesse sein können. Da eine emittierte Fluoreszenzstrahlung nur eine gewisse optische Leistung bzw. gewisse optische Intensität pro Fläche aufweist, gleichzeitig aber Bildsensoren bei einer gegebenen Pixelauflösung bzw. einer gegebenen Fläche pro Sensorpixel bei einer bestimmten Erfassungszeit bzw. Belichtungszeit nur eine bestimmte Lichtmenge erfassen können, wirkt sich je nach gewählter Erfassungszeitdauer und gegebener Pixelauflösung ein sogenanntes Sensorrauschen zu einem gewissen Grade auf das Bildsignal aus. Würde beispielsweise eine Erfassungszeitdauer mit einem relativ hohen Wert von z.B. 500 ms gewählt werden, so könnte zwar bei jedem einzelnen Sensorpixel eine gewisse Lichtmenge der Fluoreszenzstrahlung während dieser Erfassungszeitdauer gesammelt werden können und somit das hier gewonnene Nutzsignal des Bildes ein überlagertes Rauschsignal des Sensors und/oder der Elektronik zu einem hinreichenden Grad überdecken. In einem solchen Fall wäre es aber eben auch lediglich möglich, Digitalbilder zu aufeinanderfolgenden Zeitpunkten mit einem zeitlichen Abstand von 500 ms zu einander zu gewinnen, welches für eine flüssig wirkende Darstellung bzw. Auswahl eines Bildausschnittes der Probe in Abhängigkeit der Bewegungsgeste nicht ausreichen würde, da die Bildfrequenz lediglich 2 Bilder pro Sekunde betragen würde.

Dadurch, dass die Erfassungszeitdauer in dem ersten Betriebszustand kleiner gewählt wird als die Erfassungszeitdauer in dem zweiten Betriebszustand, kann die Wiederholungsfrequenz bzw. Anzeigefrequenz der Digitalbilder in dem ersten Betriebszustand hoch genug gewählt werden, um dem Nutzer den Eindruck eines flüssigen und ruckelfreien Bildes in Bezug auf seine Bewegungsgeste zu vermitteln. Ferner kann dadurch, dass in dem zweiten Betriebszustand die Erfassungszeitdauer höher gewählt wird als in dem ersten Betriebszustand, das Bildsignal für das weitere Digitalbild in hinreichender Intensität gewonnen werden und das weitere Digitalbild so in hinreichender Bildqualität dargestellt werden, so dass auch kleinere Details für den Nutzer gut erkennbar sind. Während ein Nutzer mittels der Bewegungsgeste eine Veränderung des optischen Ausschnittes der Probe, welcher in dem Digitalbild des ersten Betriebszustandes angezeigt wird, verändert, orientiert der Nutzer sich lediglich an relativ groben Strukturen, um für sich zu bestimmen, ob der gewählte Ausschnitt des Gewebes jener ist, welchen er in einer höheren Bildqualität als ein sogenanntes Standbild betrachten möchte. Mit anderen Worten: der Nutzer ist nicht daran interessiert, während der Durchführung seiner Bewegungsgeste in dem ersten Betriebszustand Digitalbilder zu den aufeinanderfolgenden Zeitpunkten zu erhalten, welche alle Details wiedergeben, sondern es reicht ihm aus, wenn diese Digitalbilder lediglich eine gewisse Mindestqualität aufweisen. Nach Beendigung seiner Bewegungsgeste benötigt er jedoch ein qualitativ höherwertigeres Bild, um alle Details genau erkennen zu können. Daher wird dann in dem zweiten Betriebszustand für das weitere Digitalbild die Fluoreszenzstrahlung mit der zweiten Erfassungszeitdauer erfasst, welche größer ist als die erste Erfassungszeitdauer des ersten Betriebszustandes.

Führt der Nutzer die Bewegungsgeste durch, so stellen sich für ihn die Digitalbilder während des ersten Betriebszustandes zu den aufeinanderfolgenden Zeitpunkten zwar aufgrund der Wahl der ersten Erfassungszeitdauer nur mit einer gewissen Bildqualität dar. Jedoch ist es für das menschliche Auge und das menschliche Gehirn während eines bewegt dargestellten Bildes nicht wirklich möglich, einen solchen Qualitätsunterschied vollständig wahrzunehmen. Daher hat der Nutzer während des ersten Betriebszustandes einen optischen Eindruck, dass die Bildqualität sich zwischen dem ersten Betriebszustand und dem zweiten Betriebszustand nur geringfügig ändert, wobei aber gleichzeitig aufgrund der erfindungsgemäßen Lösung die Digitalbilder zu den aufeinanderfolgenden Zeitpunkten während des ersten Betriebszustandes so flüssig bzw. ruckelfrei in Abhängigkeit seiner Bewegungsgeste angezeigt werden, dass der Nutzer keine merkliche Verzögerung zwischen seiner Bewegungsgeste und der Veränderung der Relativposition zwischen der optischen Einheit und der Probe wahrnimmt.

Vorteile der weiteren Ausführungsformen der Erfindung sind Gegenstand der abhängigen Ansprüche und werden in der folgenden Beschreibung unter teilweise Bezugnahme auf die Figuren näher erläutert.

Vorzugsweise zeigt wird bei Detektieren eines Beginnens einer neuen Bewegungsgeste des Nutzers der zweite Betriebszustand beendet und in den ersten Betriebszustand übergegangen.

Vorzugsweise wird das Digitalbild in dem ersten Betriebszustand und das weitere Digitalbild in dem zweiten Betriebszustand so bestimmt, dass bei gleicher Lichtintensität der Fluoreszenzstrahlung das Digitalbild des ersten Betriebszustandes und das weitere Digitalbild des zweiten Betriebszustand eine gleiche Intensität aufweisen.

Vorzugsweise wird das Digitalbild in dem ersten Betriebszustand und das weitere Digitalbild in dem zweiten Betriebszustand so bestimmt, dass bei gleicher Lichtintensität der Fluoreszenzstrahlung das Digitalbild des ersten Betriebszustandes und das weitere Digitalbild des zweiten Betriebszustand eine gleiche Intensität aufweisen, indem einer oder mehrere der folgenden Parameter für den ersten Betriebszustand und für den zweiten Betriebszustand unterschiedlich gewählt wird:
- Verstärkungsfaktor für Sensorpixelwerte,
- Anzahl von Sensorpixelwerten, welche mittels Binning zu einem Bildpixelwert zusammengefasst werden,
- Anzahl von Sensorpixelwerten, welche mittels Debayering zu einem Bildpixelwert zusammengefasst werden.

Vorzugsweise wird das Digitalbild in dem ersten Betriebszustand so bestimmt, dass es eine erste Bildauflösung aufweist, wobei das weitere Digitalbild in dem zweiten Betriebszustand so bestimmt wird, dass es eine zweite Bildauflösung aufweist, und wobei die erste Bildauflösung geringer als die zweite Bildauflösung gewählt wird.

Vorzugsweise erfolgt in dem zweiten Betriebszustand das Erfassen der emittierten Fluoreszenzstrahlung unter Verwendung mehrerer, aufeinander folgender Teil-Erfassungszeitdauern innerhalb der zweiten Erfassungszeitdauer, wobei für die Teil-Erfassungszeitdauern korrespondierende, temporäre Digitalbilder bestimmt werden und wobei das weitere Digitalbild auf Basis der temporären Digitalbilder bestimmt wird.

Vorzugsweise wird in dem zweiten Betriebszustand bei Detektieren eines Beginnens einer neuen Bewegungsgeste des Nutzers das Erfassen der emittierten Fluoreszenzstrahlung und das Bestimmen der temporären Digitalbilder abgebrochen und es wird in den ersten Betriebszustand übergegangen.

Vorzugsweise wird als Bildsensor ein Farbbildsensor verwendet.

Vorzugsweise wird als Bildsensor ein Grauwert-Bildsensor verwendet, welcher Fluoreszenzstrahlung in einem Grünkanal detektiert.

Vorzugsweise wird ferner ein weiterer Grauwert-Bildsensor verwendet, welcher Fluoreszenzstrahlung in einem Rotkanal detektiert.

Vorzugsweise wird bei einem Wechseln von dem ersten Betriebszustand in den zweiten Betriebszustand vor Einnehmen des zweiten Betriebszustandes ein Fokussieren des optischen Systems auf die Probe durchgeführt.

Im Folgenden wird die Erfindung anhand spezieller Ausführungsformen ohne Beschränkung des allgemeinen Erfindungsgedankens anhand der Figuren näher erläutert. Dabei zeigen:
**Figur 1** eine bevorzugte Ausführungsform der erfindungsgemäßen Vorrichtung,
**Figur 2a** eine Nutzereingabevorrichtung,
**Figur 2b** eine Anzeigeeinheit,
**Figur 3** eine bevorzugte Ausführungsform zur Verwendung zweier Bildsensoren,
**Figur 4** eine bevorzugte Ausführungsform eines Bildsensors mit mehreren Sensorpixeln,
**Figur 5** eine weitere bevorzugte Ausführungsform eines Bildsensors als Farbbildsensor mit mehreren Sensorpixeln,
**Figur 6** bevorzugte Schritte im Rahmen einer Durchführung einer bevorzugten Ausführungsform des erfindungsgemäßen Verfahrens,
**Figur 7** bevorzugte Schritte zur Erfassung von Fluoreszenzstrahlungen in dem zweiten Betriebszustand,
**Figur 8** unterschiedliche Bildausschnitte von Zellbildern während eines ersten Betriebszustandes,
**Figur 9** ein weiterer Ausschnitt des Zellbildes in dem zweiten Betriebszustand,
**Figur 10** unterschiedliche Bildausschnitte eines Immunfluoreszenzbildes eines Gewebes zu unterschiedlichen Zeitpunkten während des ersten Betriebszustandes,
**Figur 11** ein weiteres Digitalbild mit einem weiteren Ausschnitt des Gewebes während des zweiten Betriebszustandes.

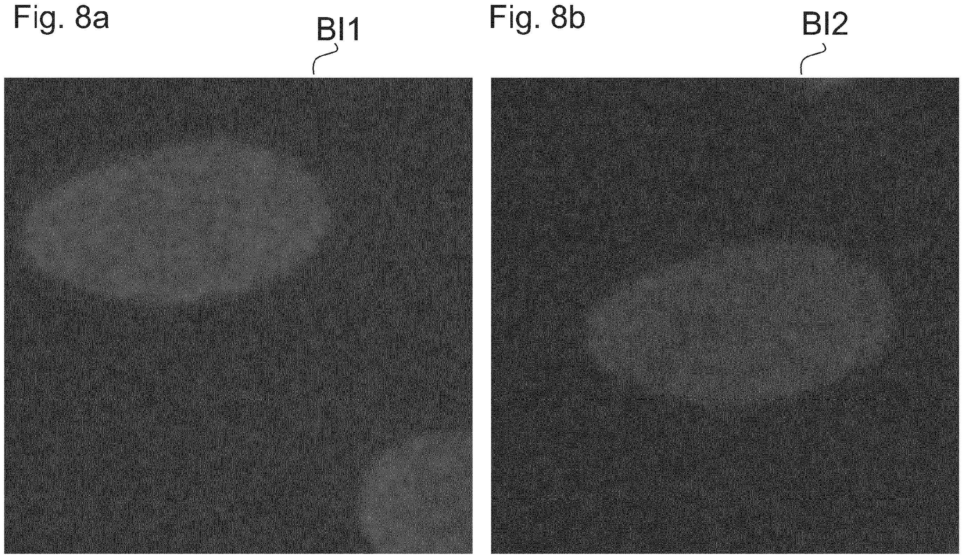

Die **Figur 8a** zeigt einen ersten Bildausschnitt BI1, in welchem sogenannte HEP-Zellen (Humane Epitheliomzellen) dargestellt sind. Dieses Bild wurde während eines ersten Betriebszustandes mit der erfindungsgemäßen Vorrichtung gewonnen. Ein Nutzer möchte beispielsweise diesen Bildausschnitt BI1 in höherer Bildqualität betrachten. Zuvor hat er möglicherweise einen anderen Bildausschnitt, BI2 aus der **Figur 8B****,** betrachtet und mittels einer Bewegungsgeste, welche über eine Nutzereingabevorrichtung eingegeben wurde, den entsprechenden Bildausschnitt so gewählt hat, wie in der **Figur 8a** gezeigt.

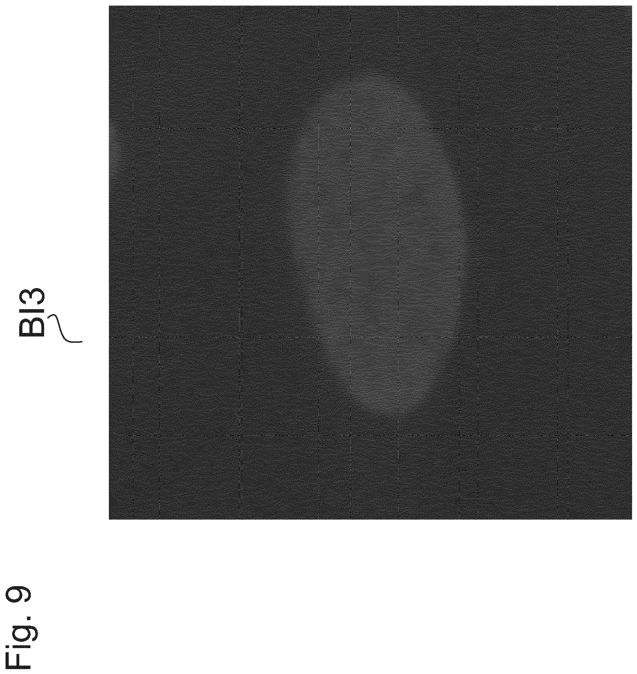

Gemäß des erfindungsgemäßen Verfahrens erfolgt dann ein Erfassen der Fluoreszenzstrahlung zur Gewinnung eines Bildes, wie z.B. des Bildes BI3 aus der **Figur 9****,** in einem zweiten Betriebszustand, welches eine höhere Bildqualität hat als die Bilder BI1, BI2 aus der **Figur 8a** bzw. **8b.**

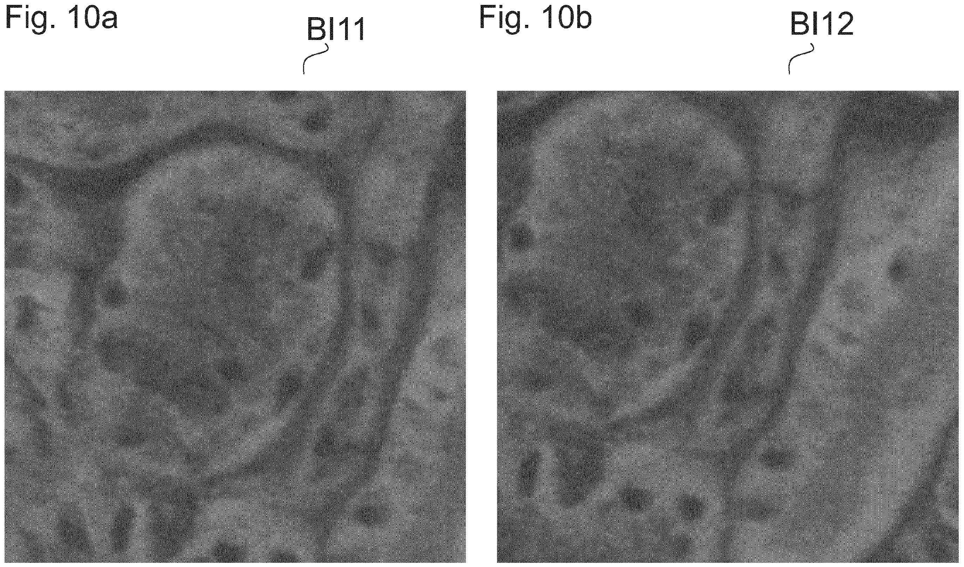

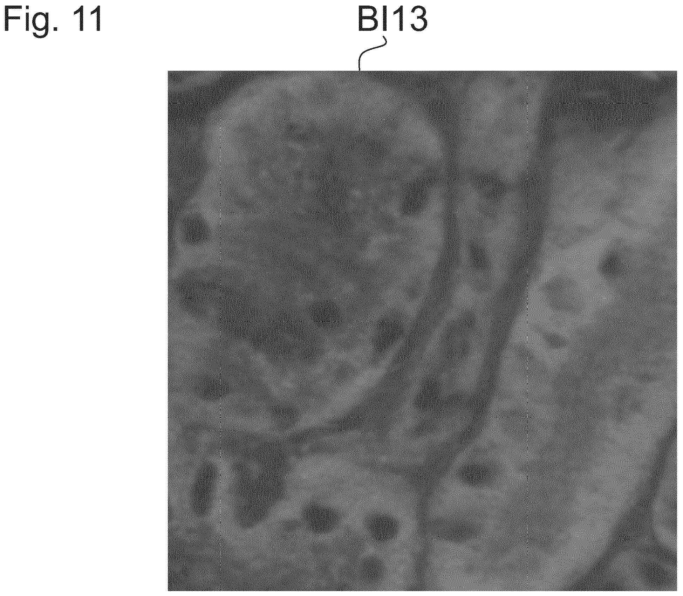

Ein weiteres Beispiel ist in den **Figuren 10a** und **10b** gegeben, bei welchen der Nutzer während des ersten Betriebszustandes von einem Bildausschnitt BI12 hin zu einem Bildausschnitt BI11 eine Relativpositionsänderung zwischen Probe und optischen System mittels Angabe seiner Bewegungsgeste herbeiführt, wobei dann nach Beendigung seiner Bewegungsgeste in dem zweiten Betriebszustand ein Bild BI13 gewonnen und angezeigt wird, wie in der **Figur 11** dargestellt.

Eine Nutzereingabevorrichtung kann beispielsweise ein sog. Touch-Screen sein, auf welchem der Nutzer durch verschieben seiner Fingerspitze auf der Touch-Screenoberfläche die Bewegungsgeste durchführt. Die Bewegungsgeste ist vorzugsweise dann beendet bzw. wird als beendet detektiert, wenn der Nutzer den Finger von der Touch-Screenoberfläche entfernt. Alternativ kann die Bewegungsgeste als beendet detektiert werden, wenn der Nutzer zeitlich gesehen eine Lageänderungen seiner Fingerspitze auf der Touch-Screenoberfläche nicht mehr bzw. nicht mehr merklich vornimmt, sodass innerhalb eines vorgegebenen Zeitfensters ein gewisser Schwellwert bezogen auf eine örtliche Änderung der Fingerspitze unterschritten wird. Der räumliche Schwellwert bezieht sich auf die Änderung der detektierten Position des Fingers auf dem Touchscreen.

Die Nutzereingabevorrichtung kann alternativ ein Eingabemittel wie beispielsweise eine Computermaus sein, über welche der Nutzer eine solche Bewegungsgeste eingeben kann. Abhängig von einem durch die Nutzereingabevorrichtung erfassten Eingabesignal kann dann eine Steuereinheit eine Bewegungsgeste ableiten bzw. detektieren.

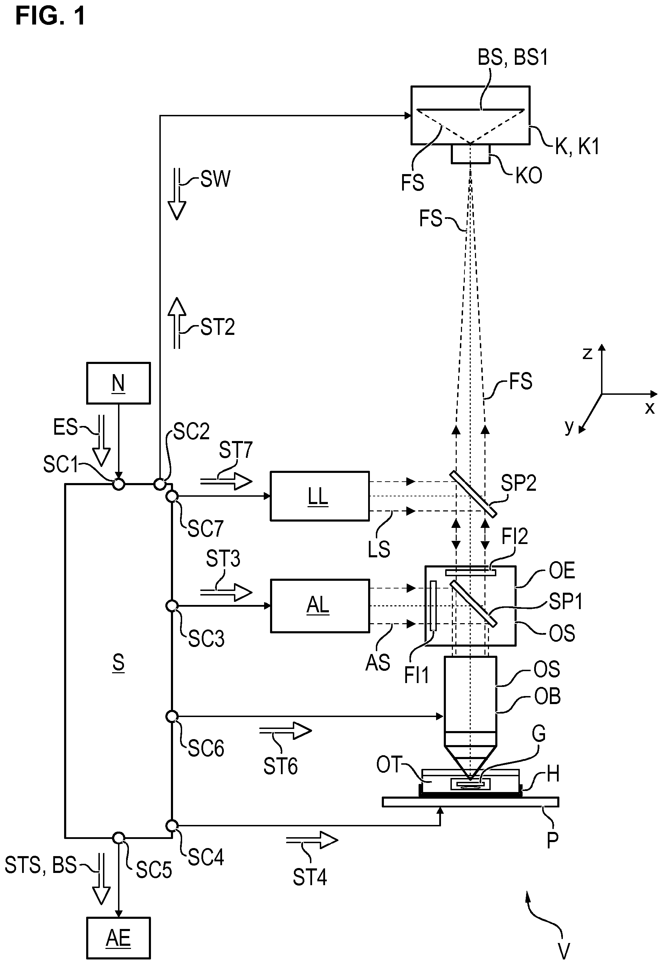

Die **Figur 1** zeigt eine bevorzugte Ausführungsform der erfindungsgemäßen Vorrichtung V, welche zur Durchführung des erfindungsgemäßen Verfahrens ausgebildet ist. Eine Probe G, vorzugsweise eine Gewebeprobe, ist beispielsweise innerhalb eines Objektträgers OT bereitgestellt. Eine Halterungsvorrichtung H dient der Halterung des Objektträgers OT bzw. der Probe G. Die Halterungsvorrichtung H ist vorzugsweise mit einer Positioniereinheit P gekoppelt, welche ausgebildet ist, eine Relativposition zwischen der Probe G und einem optischen System OS zu verändern. Die Änderung der Relativposition zwischen der Probe G bzw. der Halterungsvorrichtung H und dem optischen System OS kann alternativ zu der in der **Figur 1** dargestellten Variante auch dadurch herbeigeführt werden, dass das optische System OS durch eine Positioniereinheit in seiner Relativposition gegenüber dem Gewebe G bzw. der Halterung H geändert wird.

Das optische System OS führt eine Fluoreszenzstrahlung FS von der Probe G hin zu einem Bildsensor BS. Das optische System OS besteht vorzugsweise aus einem Objektiv OB und einer weiteren optischen Einheit OE, auf welche im Weiteren noch genauer eingegangen wird.

Der Bildsensor BS weist mehrere Sensorpixel zum Erfassen von von der Probe G emittierter Fluoreszenzstrahlung FS auf. Ein Anregungslicht bzw. eine Anregungsstrahlung AS wird mittels einer Anregungslichtquelle AL bereitgestellt und hierdurch die Probe G beleuchtet. Die Anregungsstrahlung AS passiert zunächst einen optischen Filter FI1, welcher die gewünschte Anregungsstrahlung in einem bestimmten Wellenlängenbereich herausfiltert. Die Strahlung AS wird dann über einen dichroitischen Spiegel SP1 hin zu dem Objektiv OB bzw. der Probe G geleitet.

Die von der Probe G emittierte Fluoreszenzstrahlung FS wird durch das Objektiv OB zurück zu dem dichroitischen Spiegel geleitet, welcher die Fluoreszenzstrahlung mit ihrer Wellenlänge, welche sich von der Wellenlänge der Anregungsstrahlung AS unterscheidet, hin zu dem Bildsensor führt. Ein Filter FI2 filtert vorzugsweise die Fluoreszenzstrahlung heraus. Die Fluoreszenzstrahlung FS passiert hierbei vorzugsweise ein Kameraobjektiv KO, welches dann die Fluoreszenzstrahlung FS bis hin zu dem Bildsensor BS einer Kamera K leitet.

Die erfindungsgemäße Vorrichtung V weist wenigstens eine Steuereinheit S auf. Die Steuereinheit S wiederum weist eine erste Schnittstelle SC1 hin zu einer Nutzereingabevorrichtung N auf. Über die Schnittstelle SC1 nimmt die Steuereinheit S ein Eingabesignal ES der Nutzereingabevorrichtung N entgegen und kann hieraus eine Bewegungsgeste eines Nutzers ableiten bzw. bestimmen und sowohl eine Beendigung der Bewegungsgeste als auch ein Beginnen einer neuen Bewegungsgeste detektieren.

Über eine Schnittstelle SC5 kann die Steuereinheit S ein Bildsignal BS bzw. ein Steuersignal ST5 hin zu einer Anzeigeeinheit AE ausgeben und so Digitalbilder anzeigen lassen. Die Nutzereingabevorrichtung N und die Anzeigeeinheit AE können auch in einer einzigen, kombinierten Einheit wie beispielsweise einem Touch-Screen gegeben sein. Die Anzeigeeinheit kann alternativ ein sogenannter Computermonitor sein, wobei dann die Nutzereingabevorrichtung N eine so genannte Computermaus sein kann. Sind die Nutzereingabevorrichtung N und die Anzeigeeinheit AE vereint, so können auch die Schnittstellen SC1, SC5 als eine vorzugsweise bidirektionale Schnittstelle vereint sein.

Die Steuereinheit S nimmt über eine Schnittstelle SC2 Sensorpixelwerte SW des Bildsensors BS entgegen. Ferner kann die Steuereinheit S über die Schnittstelle SC2 ein oder mehrere Steuersignale ST2 an die Kamera K übermitteln. Ein solches Steuersignal ST2 kann beispielsweise eine zu verwendende Erfassungszeit zur Erfassung von Fluoreszenzstrahlung durch den Bildsensor BS bzw. Kamera K indizieren. Ferner kann ein solches Steuersignal ST2 auch ein sogenanntes Triggersignal sein, um ein Erfassen von Fluoreszenzstrahlen durch den Bildsensor BS zu einem bestimmten Zeitpunkt anzufordern.

Vorzugsweise weist die Steuereinheit S eine Schnittstelle SC6 auf, über welche die Steuereinheit S mittels eines Steuersignals ST6 das Objektiv OB bzw. das optische System OS ansteuert, um eine optische Einstellung wie z.B. eine Fokussierung des optischen Systems OS zu verändern.

Über eine Schnittstelle SC3 kann die Steuereinheit S mittels eines Steuersignals ST3 die Anregungslichtquelle AL ansteuern, um das Anregungslicht ein- oder auszuschalten.

Ferner kann die Steuereinheit S über die Schnittstelle SC7 mittels eines Steuersignals ST7 die Laserlichtquelle LL ein oder ausschalten und vorzugsweise ferner dessen Helligkeit ändern.

Die Positioniereinheit P ist ausgebildet, zumindest eine Lateralposition der Probe in XY-Richtung gegenüber dem optischen System OS zu verändern. Die Steuereinheit S kann über die Schnittstelle SC4 mittels des Ansteuersignals ST4 die Positioniereinheit P so ansteuern, dass eine gewünschte Änderung der Relativposition zwischen der Probe G bzw. der Halterung H gegenüber dem optischen System OS bewirkt wird.

Vorzugsweise ist die Positioniereinheit P auch noch ausgestaltet, eine Z-Position zur Veränderung eines Abstandes zwischen der Probe G und dem optischen System OS zum Zweck eine Fokussierung zu verändern. Hierzu kann insbesondere Laserstrahlung LS verwendet werden, welche über eine Laserlichtquelle LL dann vorzugsweise unter Verwendung eines dichroitischen Spiegels SP2 in das optische System OS eingekoppelt werden kann und welche dann in dem Bildsensor BS erfasst werden kann. In einer alternativen Ausgestaltung ist das Objektiv ausgebildet, in Abhängigkeit des Steuersignals ST6 einen Abstand in Z-Richtung zwischen dem Objektiv und der Probe G zu ändern, um eine Fokussierungseinstellung zu verändern.

Gemäß der hier gezeigten Ausführungsform ist der Bildsensor BS ein Farbbildsensor BS1, sodass die Kamera K eine Farbkamera K1 ist.

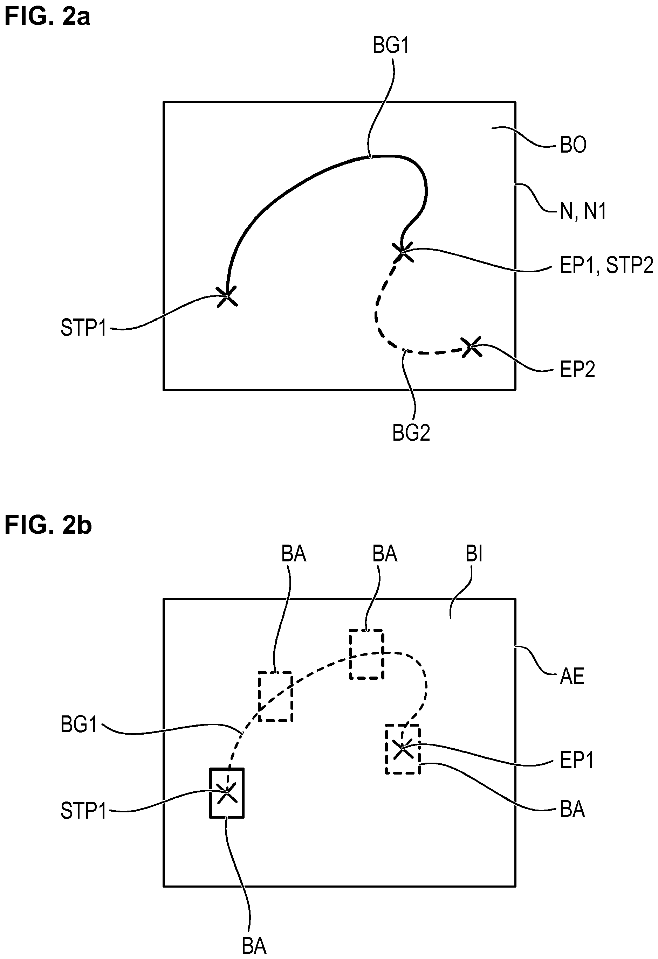

Die **Figur 2a** zeigt eine Darstellung einer Nutzereingabevorrichtung N in Form eines Touchscreens bzw. einer berührungsempfindlichen Anzeigeeinheit N1. Ein Nutzer kann beispielsweise auf der berührungsempfindlichen Oberfläche BO eine Bewegungsgeste BG1 durchführen, indem er seinen Finger an einem Startpunkt STP1 aufsetzt und den Finger entlang der Kurve der Bewegungsgeste BG1 bis hin zu einem Endpunkt EP1 führt und dann dort die Bewegungsgeste beendet. Ein solches Beenden einer Bewegungsgeste kann dann durch die Steuereinheit S aus der **Figur** 1 detektiert werden.

**Figur 2b** zeigt eine Anzeigeeinheit AE, welche vorzugsweise identisch mit der Nutzereingabevorrichtung N1 der **Figur 2a** ist. Alternativ kann die Anzeigeeinheit AE in dem Fall, dass die Nutzereingabevorrichtung eine Computermaus ist, ein sogenannter Computermonitor bzw. Bildschirm sein.

Auf der Anzeigeeinheit ist zur Orientierung des Lesers in gestrichelter Linie wiederum die Bewegungsgeste BG1 dargestellt. Ein bestimmter Bildausschnitt BA aus dem Immunfluoreszenzbild bzw. der Probe wird dann aufgrund der Änderung der Relativposition zwischen dem Positioniersystem bzw. der Positioniereinheit P und dem optischen System OS aus der Figur 1 entlang der Kurve der Bewegungsgeste BG1 bis hin zu dem Endpunkt EP1 verschoben. Beendet der Nutzer die Bewegung an diesem Endpunkt EP1, so wird dann in den zweiten Betriebszustand gewechselt und dort ein Immunfluoreszenzbild unter Verwendung der zweiten Erfassungszeitdauer bestimmt.

Die **Figur 2a** zeigt ferner eine weitere Bewegungsgeste BG2, welche der Nutzer von einem Startpunkt STP2, welcher mit dem vorigen Endpunkt EP1 zusammenfällt, dann bis hin zu einem weiteren Endpunkt EP2 ausführt. Wird ein solches Beginnen einer neuen Bewegungsgeste BG2 des Nutzers detektiert, so wird dann vorzugsweise der zweite Betriebszustand beendet und wieder in den ersten Betriebszustand übergegangen.

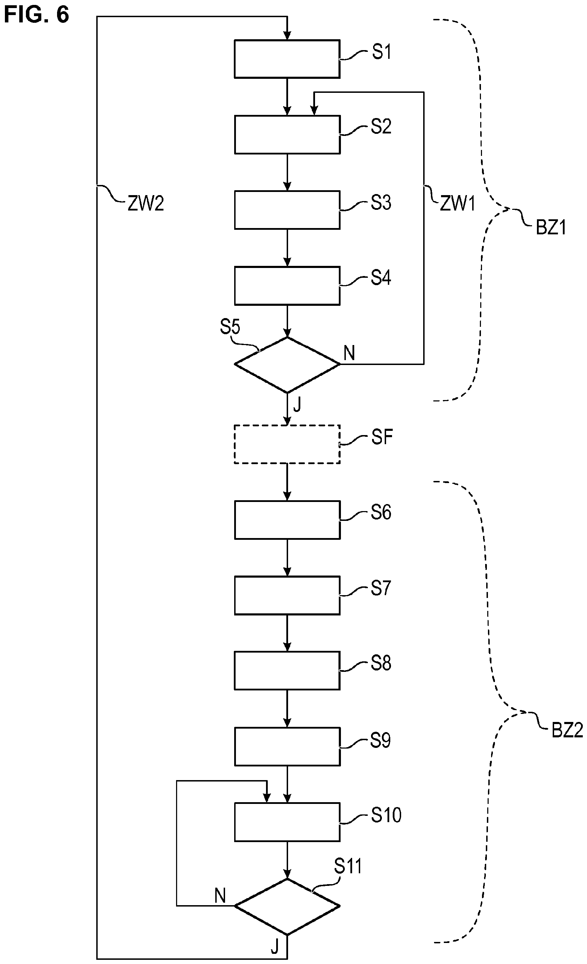

Die **Figur 6** zeigt vorzugsweise durchzuführende Schritte gemäß einer Ausführungsform des vertragsgemäßen Verfahrens. Während des ersten Betriebszustandes BZ1 erfolgt ein Schritt S1, in welchem die Anregungslichtquelle eingeschaltet wird, sodass die Probe während des ersten Betriebszustandes fortdauernd mit der Anregungsstrahlung beleuchtet wird. Vorzugsweise werden in dem Schritt S1 zudem von der Steuereinheit Konfigurationsparameter über die Schnittstelle SC2 an die Kamera K übertragen.

In einem Schritt S2 erfolgt dann eine Veränderung der Relativposition zwischen der Probe und dem optischen System in Abhängigkeit einer detektierten Bewegungsgeste des Nutzers. Hierbei kann die Veränderung der Relativposition derart erfolgen, dass zunächst nur ein Teil einer Bewegungsgeste detektiert wird und dann entsprechend dieser Teilbewegungsgeste die Relativposition zu einem gewissen Teil verändert wird.

In einem Schritt S3 erfolgt dann ein Erfassen der Fluoreszenzstrahlung unter Verwendung der ersten Erfassungszeitdauer. Diese wird so gewählt, dass eine hinreichend flüssige Bewegung der zu den aufeinander folgenden Zeitpunkten angezeigten Digitalbilder gegeben ist. Auf Basis der für die einzelnen Sensorpixel sich ergebenden Sensorpixelwerte wird dann in dem Schritt S3 durch die Steuereinheit ein Digitalbild bestimmt.

In einem Schritt S4 wird dann das Digitalbild mittels Ausgabe an die Anzeigeeinheit angezeigt.

In einem Schritt S5 kann dann überprüft werden, ob die Bewegungsgeste des Nutzers durch den Nutzer beendet wurde. Ist dies nicht der Fall, so wird in dem ersten Betriebszustand BZ1 verblieben. Dieses ist durch die Verzweigung ZW1 in der Figur 6 dargestellt.

Es erfolgt also in dem ersten Betriebszustand BZ1 ein Erfassen der Fluoreszenzstrahlungen sowie das Bestimmen und das Anzeigen des Digitalbildes zu aufeinanderfolgenden Zeitpunkten mit einer bestimmten Wiederholungsfrequenz.

Wird allerdings in dem Schritt S5 detektiert, dass die Bewegungsgeste durch den Benutzer beendet wurde, so wird von dem ersten Betriebszustand BZ1 in den zweiten Betriebszustand BZ2 gewechselt.

Vorzugsweise wird zwischen dem ersten Betriebszustand BZ1 dem zweiten Betriebszustand BZ2 noch in einem optionalen Zwischenschritt SF vor Einnehmen des zweiten Betriebszustandes BZ2 ein Fokussieren des optischen Systems auf die Probe unter Verwendung von Laserstrahlung LS der Laserlichtquelle LL aus der **Figur 1** durchgeführt. Die Laserstrahlung LS wird vorzugsweise mittels eines dichroitischen Spiegels SP2 eingekoppelt. Ein solches Autofokussierverfahren unter Verwendung von Laserlichtstrahlung LS ist beispielsweise in der EP-Patentanmeldung Nr. 17 001 037.5 der Anmelderin detailliert beschrieben. Während des Vorgangs des Autofokussierens ist vorzugsweise die Anregungslichtquelle zum Aussenden der Anregungsstrahlung ausgeschaltet, wobei ferner vorzugsweise während des Vorgangs des Autofokussierens das in dem ersten Betriebszustand zuletzt bestimmte Bild fortdauernd auf der Anzeigeeinheit angezeigt wird. Somit wird während des Autofokussierens der Eindruck einer "Live-Mikroskopie" für den Nutzer nicht unterbrochen.

In dem zweiten Betriebszustand BZ2 erfolgt in einem Schritt S6 ein Beleuchten der Probe mit der Anregungsstrahlung bzw. ein Einschalten der Anregungslichtquelle zum Beleuchten der Probe mit der Anregungsstrahlung.

In einem Schritt S7 erfolgt dann das Erfassen der Fluoreszenzstrahlung mittels der mehreren Sensorpixel unter Verwendung der zweiten Erfassungszeitdauer, welche größer als die erste Erfassungszeitdauer ist. Auf Basis resultierender Sensorpixelwerte des Bildsensors erfolgt dann ein Bestimmen eines weiteren Digitalbildes, welches dann in einem Schritt S8 angezeigt wird. In einem Schritt S9 erfolgt ein Beenden des Erfassens der Fluoreszenzstrahlung und ein Beenden des Beleuchtens der Probe nach Ablauf der zweiten Erfassungszeitdauer. Das Anzeigen des weiteren Digitalbildes erfolgt in einem Schritt S10 fortdauernd für den zweiten Betriebszustand.

Dadurch, dass in dem zweiten Betriebszustand BZ2 nach dem Ablauf der zweiten Erfassungszeitdauer ein weiteres Digitalbild fortdauernd angezeigt wird und dass ferner das Beleuchten der Probe durch die Anregungsstrahlung beendet wird, wird ein sogenanntes Ausbrennen der Probe aufgrund eines Beleuchtens mit der Anregungsstrahlung minimiert. Für den Nutzer ergibt sich ferner durch das erfindungsgemäße Verfahren der Eindruck, dass er nicht nur während des Durchführens seiner Bewegungsgeste fortdauernd erfasste Bilder als Repräsentationen eines realen Mikroskopbildes angezeigt bekommt, sondern dass auch während des zweiten Betriebszustandes weiterhin ein fortdauerndes Mikroskopbild angezeigt wird, da er das weitere Digitalbild fortdauernd angezeigt bekommt. Durch die hier vorgeschlagene Lösung wird also erreicht, dass der Nutzer den Eindruck einer fortdauernden Mikroskopie erhält, obwohl keine weiteren Bilder mit gleichzeitiger Beleuchtung des Gewebes durch die Anregungsstrahlung gewonnen werden.

In einem Schritt S11 erfolgt dann ein Überprüfen, ob eine neue Bewegungsgeste des Nutzers detektiert wird. Dies kann beispielsweise die Bewegungsgeste BG2 aus der **Figur 2A** **sein.**

Ist dies nicht der Fall, so wird zu dem Schritt S10 zurückgekehrt und weiterhin fortdauernd das weitere Digitalbild in dem zweiten Betriebszustand BZ2 angezeigt. Ist dies jedoch der Fall, so wird der zweite Betriebszustand beendet und übergegangen in den ersten Betriebszustand BZ1. Dieses ist durch die Verzweigung ZB2 in der **Figur 6** dargestellt.

Hierdurch wird erreicht, dass der Nutzer durch seine erste Bewegungsgeste BG1 aus der **Figur 2a** zunächst einen ersten Bildausschnitt in das angezeigte Bild bewegen kann, um diesen Bildausschnitt nach Beendigung der Bewegungsgeste in dem zweiten Betriebszustand in hoher Bildqualität angezeigt zu bekommen. Der Nutzer kann aber ferner durch Beginnen der zweiten Bewegungsgeste in den ersten Betriebszustand zurückkehren und mittels der zweiten Bewegungsgeste einen neuen Bildausschnitt wählen.

In der hier unter Bezug auf die Figur 6 beschriebenen bevorzugten Ausführungsform des vorgeschlagenen Verfahrens erfolgt das Verändern der Relativposition in Schritt S2, das Erfassen der Fluoreszenzstrahlung in dem Schritt S3 und das Anzeigen des Digitalbildes in dem Schritt S4 in der Form, dass die Schritte S2, S3 und S4 alle seriell aufeinander folgen. In einer alternativen Ausführungsform kann das Verändern der Relativposition fortlaufend durchgeführt werden, während zeitlich parallel zu dem Verändern der Relativposition das Erfassen der Fluoreszenzstrahlung und das Anzeigen des Digitalbildes erfolgt. Es würden dann beispielsweise die Schritte S3 und S4 aufeinander zeitlich folgen, diese Sequenz der Schritte S3 und S4 aber parallel zu dem Schritt S2 ausgeführt werden.

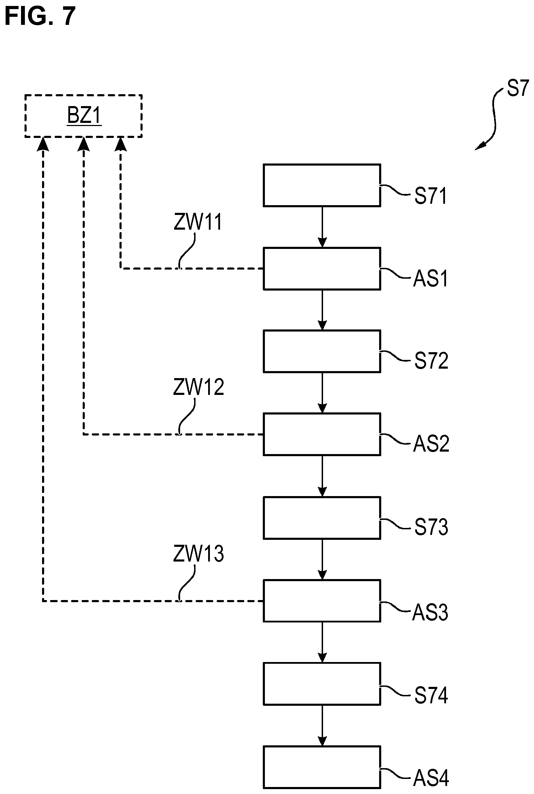

In der **Figur 7** ist eine Variante des Schrittes S7 zum Erfassen der Fluoreszenzstrahlung in dem zweiten Betriebszustand dargestellt, bei welcher temporäre Digitalbilder für jeweilige Teilerfassungszeitdauern bestimmt werden, wobei ferner das anzuzeigende weitere Digitalbild des zweiten Betriebszustandes auf Basis der temporären Digitalbilder bestimmt wird.

Die Schritte S71 bis AS4 werden in den zweiten Betriebszustand während der zweiten Erfassungszeitdauer zum Erfassen der emittierten Fluoreszenzstrahlung durchgeführt.

Zunächst wird in einem Schritt S71 während einer Teilerfassungszeitdauer von beispielsweise 19 ms ein erstes temporäres Digitalbild bestimmt und in einem Anzeigeschritt AS1 angezeigt. In einem nächsten Verfahrensschritt S72 wird dann ein weiteres temporäres Digitalbild unter Verwendung einer Teilerfassungszeitdauer von 19 ms bestimmt und das weitere Digitalbild als Mittelwert der beiden temporären Digitalbilder aus den Schritten S71 und S72 bestimmt und in dem Schritt AS2 angezeigt. Während eines Erfassungsschrittes S73 erfolgt dann ein Erfassen der emittierten Fluoreszenzstrahlung unter Verwendung der Teilerfassungszeitdauer von 19 ms zur Gewinnung bzw. Bestimmung eines dritten temporären Digitalbildes, wobei das weitere Digitalbild dann aus den drei temporären Digitalbildern mittels Mittelung der Pixelwerte bestimmt wird. Das weitere Digitalbild wird dann in dem dritten Anzeigeschritt AS3 angezeigt. Entsprechendes erfolgt dann in einem Erfassungsschritt S74 und einem Anzeigeschritt AS4, sodass in diesem Ausführungsbeispiel vier temporäre Digitalbilder bestimmt werden und das weitere Digitalbild auf Basis der temporären Digitalbilder bestimmt wird und dann angezeigt wird. In diesem Ausführungsbeispiel besteht die zweite Erfassungszeitdauer aus vier Teilerfassungszeitdauern.

Ein solches Verfahren zur Bestimmung des weiteren Digitalbildes auf Basis mehrerer temporärer Digitalbilder ist vorteilhaft, da ein Sensorrauschen bzw. ein den Bildsignalen überlagertes Rauschsignal aufgrund seiner statistischen Eigenschaften durch die Mittelung der temporären Digitalbilder reduziert bzw. minimiert wird, gleichzeitig aber die tatsächliche Intensität bzw. Signalintensität der Fluoreszenzstrahlung als Nutzsignal erhalten bleibt.

Verzweigungen ZW11, ZW12, ZW13 von den korrespondierenden Schritten AS1, AS2, AS3 weg illustrieren jeweils ein mögliches Abbrechen des Bestimmens der temporären Digitalbilder in dem Fall, dass in dem zweiten Betriebszustand ein Beginn einer neuen Bewegungsgeste des Nutzers detektiert wird. Es wird dann auch das Erfassen der emittierten Fluoreszenzstrahlung in dem zweiten Betriebszustand abgebrochen und es wird in den ersten Betriebszustand BZ1 übergegangen. Diese Ausgestaltung ist vorteilhaft, da es dem Nutzer somit möglich ist, bereits während der Mittelung der temporären Digitalbilder eine neue Bewegungsgeste zu beginnen, bevor die gesamte aus Teilerfassungszeitdauern akkumulierte zweite Erfassungszeitdauer abgelaufen ist. Würde das weitere Digitalbild in dem zweiten Betriebszustand unter Verwendung einer einzigen, gesamten, ununterbrochenen zweiten Erfassungszeitdauer gewonnen werden, so könnte der Nutzer eine Änderung der Relativposition bzw. ein Auswählen eines Bildausschnittes durch Beginn einer neuen Bewegungsgeste erst dann herbeiführen, wenn die gesamte zweite Erfassungszeitdauer abgelaufen ist. Gemäß der hier vorzugsweisen Lösung aus der **Figur 7** kann der Nutzer aber aufgrund des durchgeführten Mittelungsvorgangs der temporären Digitalbilder und der Möglichkeit des Abbruchs des zweiten Betriebszustandes bereits vor Ablauf des zweiten Erfassungszeitraumes die Änderung der Relativposition bzw. die Wahl des Bildausschnittes verändern. Das weitere Digitalbild wird fortlaufend aus den temporären Digitalbildern bestimmt und seine Darstellung auf der Anzeigeeinheit nach jeder der entsprechenden aktualisiert; hierdurch wird dem Nutzer bereits vor Ablauf der gesamten zweiten Erfassungszeitdauer ein Bild von einer Bildqualität angezeigt, welche besser als die Bildqualität eines Digitalbildes während des ersten Betriebszustandes ist. Insbesondere würde der Nutzer so fortlaufend während des zeitlichen Verlaufes ein hinsichtlich seiner Bildqualität kontinuierlich verbesserndes Bild angezeigt bekommen, bis schließlich die zweite Erfassungszeitdauer abgelaufen ist.

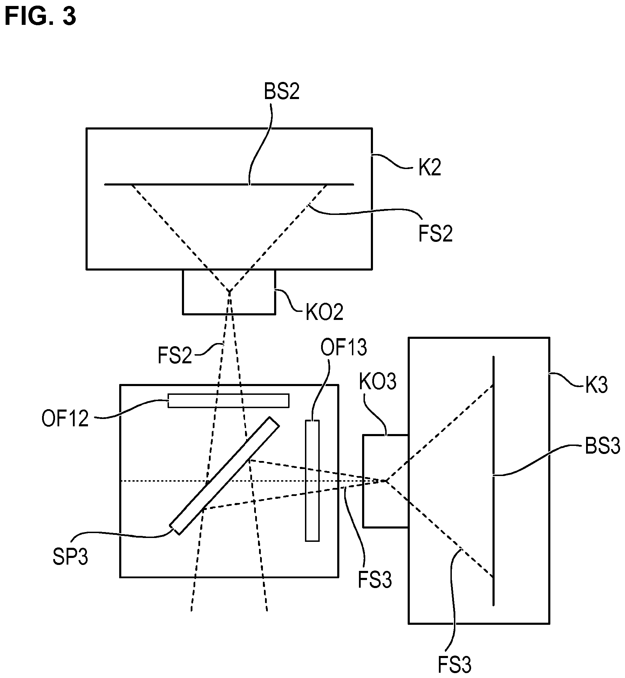

Gemäß der **Figur 1** kann als Kamera K eine sogenannte Farbkamera K1 mit einem Farbbildsensor BS1 als Bildsensor BS verwendet werden. Gemäß einer alternativen Ausführungsform aus der Figur 3 kann als Kamera K eine Kamera K2 mit einem Grauwert-Bildsensor BS2 verwendet werden, welche die Fluoreszenzstrahlung FS, FS2 in einem Grünkanal detektiert. Hierzu kann mittels eines optischen Filters OF12 ein sogenannter Grünkanal der Fluoreszenzstrahlung FS2 selektier werden und dann dem Grauwert-Bildsensor BS2 zugeführt werden. Dieses erfolgt dann vorzugsweise wieder über ein Kameraobjektiv KO2.

Gegenüber der Verwendung eines einzigen Grauwert-Bildsensors birgt die Verwendung eines Farbbildsensors den Vorteil, dass nicht nur eine Fluoreszenzstrahlung aufgrund der Fluoreszenz des zuvor eingebrachten Fluoreszenzfarbstoffes in dem Bild sichtbar wird, sondern dass auch eine sogenannte Autofluoreszenz des Gewebes in einem weiteren Kanal, vorzugsweise in einem Rotkanal, erfasst und dargestellt werden kann. Dieses können beispielsweise Färbungen mit einem Braunanteil sein. Eine solche zusätzliche Bildinformation kann durch die Verwendung eines Farbbildsensors dem Nutzer zusätzliche optische Informationen hinsichtlich des dargestellten Gewebes bereitstellen.

Gemäß einer vorzugsweisen Ausführungsform gemäß der **Figur 3** kann die Vorrichtung ferner neben dem Grauwert-Bildsensor BS2 einen weiteren Grauwert-Bildsensor BS3 aufweisen, welcher vorzugsweise Fluoreszenzstrahlung in einem Rotkanal detektiert. Hierzu kann die Fluoreszenzstrahlung FS des Gewebes mittels des dichroitischen Spiegels SP3 so aufgeteilt werden, dass die Fluoreszenzstrahlung FS3 den Rotkanal repräsentiert. Hierbei kann ein optischer Filter OF13 den Rotkanal selektieren bzw. durchlassen. Dann kann die Information bzw. Farbinformation der beiden Grauwert-Bildsensoren BS2 und BS3 durch die Steuereinheit S überlagert bzw. zusammengeführt werden.

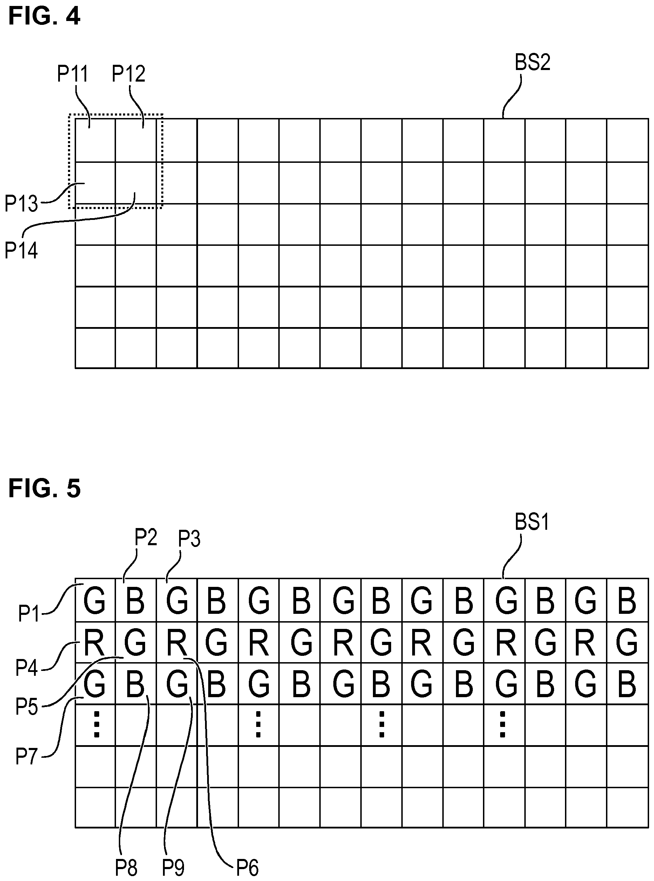

Die **Figur 4** zeigt einen Grauwert-Bildsensor BS2, welcher Pixel P11, P12, P13, P14 aufweist. Der Grauwert-Bildsensor BS2 kann mit seiner Pixelauflösung bzw. Pixelanzahl pro Fläche einem sog. "Binning" unterzogen werden, indem die Pixelwerte der Pixel P12, P12, P13, P14 aufsummiert werden, wobei dann für ein einzelnes Bildpixel BP eine vierfacher Fläche gegenüber einer Fläche eines Sensorpixel verwendet wird. Hierdurch ergibt sich eine Reduktion der Auflösung in dem resultierenden Digitalbild gegenüber der Auflösung des Bildsensors BS2 um einen Faktor 4.

Die **Figur 5** zeigt ein Beispiel eines Farbbildsensors BS1, welcher unterschiedliche Pixel P1,..., P9 aufweist, wobei die entsprechenden Buchstaben G, B, R jeweils angeben, ob ein Grün (G), Blau (B) oder Rot (R) Farbfilter vor dem jeweiligen Pixel P1,.. P9 vorhanden ist. Für das Farbpixel P5 kann dann eine RGB-Farbbildinformation mit drei Farbkanälen beispielsweise dadurch gewonnen werden, dass ein sog. "De-Bayering" durchgeführt wird. Hierbei wird für das Pixel P5 eine Rotinformation auf Basis der Pixelwerte der Pixel P4 und P6 gewonnen, eine Grüninformation auf Basis der Sensorpixelwerte der Pixel P1, P3, P5, P7 und P9 sowie eine Blauinformation auf Basis der Pixelwerte der Pixel P8 und P2. Hierdurch lässt sich also für jedes einzelne Pixel P1,..., P9 eine dreikanalige Farbinformation als Rot-, Grün- und Blaukanal gewinnen, ohne eine Auflösungsreduktion in dem resultierenden Bild hinsichtlich der Pixelanzahl bzw. Pixeldichte pro Fläche gegenüber dem Bildsensor bewirkt wird. Würde man eine auf Basis der Sensorpixel P1, ..., P9 gewonnene RGB-Information in einem einzelnen Bildpixel zusammenfassen, welches der Fläche aller der Sensorpixel P1, ..., P9 entspricht, so entspräche dies neben dem Effekt des De-Bayerings zusätzliche einer Auflösungsreduktion, nämlich um den Faktor 9. Würde man entsprechend die Sensorpixel P1, P2, P3, P4 des Sensors BS1 aus der Figur 5 durch ein De-Bayering nicht nur zum Gewinnen einer RGB-Information verwenden sondern auch flächenmäßig zusammenfassen, so würde durch ein solches De-Bayering eine Auflösungsreduktion um einen Faktor 4 bewirkt werden, ähnlich einem Binning der Sensorpixel P11, ..., P14 aus Figur 4.

Zur Bestimmung von Bildpixelwerten eines Digitalbildes aus Sensorpixelwerten können die Sensorpixelwerte auch durch einen Verstärker um einen Verstärkungsfaktor erhöht werden, wobei allerdings eine solche Verstärkung nicht nur das Nutzsignal des Sensorpixel erhöht sondern auch ein Rauschsignal wie z.B. ein Sensorrauschen.

Zusammengefasst kann festgestellt werden, dass abhängig von der Wahl des Bildsensors, welcher ein Grauwert-Sensor oder ein Farbsensor sein kann, und abhängig von einer anzustrebenden Bildwiederholungsfrequenz unterschiedliche Parameter unterschiedlich gewählt werden können, um ein Digitalbild aus den Sensorpixelwerten zu bestimmen:
- Verstärkungsfaktor für Sensorpixelwerte,
- Anzahl von Sensorpixelwerten, welche mittels Binning zu einem Bildpixelwert zusammengefasst werden,
- Anzahl von Sensorpixelwerten, welche mittels Debayering zu einem Bildpixelwert zusammengefasst werden.

Vorzugsweise werden das Digitalbild in dem ersten Betriebszustand und das weitere Digitalbild in dem zweiten Betriebszustand so bestimmt, dass bei gleicher Lichtintensität der Fluoreszenzstrahlung das Digitalbild des ersten Betriebszustandes und das weitere Digitalbild des zweiten Betriebszustand eine gleiche Intensität aufweisen.

Vorzugsweise wird hierbei das Digitalbild in dem ersten Betriebszustand so bestimmt, dass es eine erste Bildauflösung aufweist, wobei das weitere Digitalbild in dem zweiten Betriebszustand so bestimmt wird, dass es eine zweite Bildauflösung aufweist, und wobei die erste Bildauflösung geringer als die zweite Bildauflösung gewählt wird.

Im Folgenden werden Beispiele angeführt, welche es dem Fachmann erlauben, die unterschiedlichen Parameter so zu wählen, dass eine oder mehrere Ausführungsformen des hier beschriebenen Verfahrens erreicht werden.

In einem ersten Beispiel sei angenommen, dass nur ein Grauwert-Bildsensor mit einer Sensorauflösung von 2448 x 2048 Pixel verwendet wird. In dem ersten Betriebszustand können dann jeweils 16 Pixel gemeinsam per Binning zur Gewinnung eines Bildpixels zusammengefasst werden. Die erste Erfassungszeitdauer kann dann 19 ms betragen, so dass ungefähr eine Bildwiederholrate von 53 Bildern pro Sekunde erreicht wird. Es wird vorzugsweise keine Verstärkung der Sensorpixelwerte vorgenommen. Das resultierende Bild hat dann in dem ersten Betriebszustand eine Auflösung von 615 x 512 Pixel. In dem zweiten Betriebszustand können dann als zweite, durchgehende Erfassungszeitdauer 300 ms verwendet werden, was lediglich einer Bildwiederholrate von 3 Bildern pro Sekunde entsprechen würde. Es wird auch hier vorzugsweise keine Verstärkung der Sensorpixelwerte und auch kein Binning durchgeführt. Das resultierende Bild hat dann in dem ersten Betriebszustand eine Auflösung von 2448 x 2048 Pixel. Die Intensitätswerte wären dann in dem ersten und dem zweiten Betriebszustand des ersten Beispiels bei gleicher Intensität der Fluoreszenzstrahlung gleich.

In einem zweiten Beispiel sei angenommen, dass ein Farb-Bildsensor mit einer Sensorauflösung von 2448 x 2048 Pixel verwendet wird. In dem ersten Betriebszustand können dann jeweils 4 Pixel gemeinsam per De-Bayering zur Gewinnung eines Bildpixels mitsamt einer Auflösungsreduktion um einen Faktor 4 zusammengefasst werden. Die erste Erfassungszeitdauer kann dann 19 ms betragen, so dass ungefähr eine Bildwiederholrate von 53 Bildern pro Sekunde erreicht wird. Es wird vorzugsweise eine Verstärkung der Sensorpixelwerte um einen Linearfaktor 300/19 vorgenommen. Das resultierende Bild hat dann in dem ersten Betriebszustand eine Auflösung von 1224 x 1024 Pixel. In dem zweiten Betriebszustand können dann als eine durchgehende zweite Erfassungszeitdauer 300 ms verwendet werden, was lediglich einer Bildwiederholrate von 3 Bildern pro Sekunde entsprechen würde. Es wird auch hier vorzugsweise keine Verstärkung der Sensorpixelwerte vorgenommen. Jeweils neun Sensorpixelwerte werden dann mittels De-Bayering zur Bestimmung einer RGB-Information eines einzelnen Pixels zusammengefasst, ohne eine Auflösungsreduktion herbeizuführen. Das resultierende Bild hat dann in dem ersten Betriebszustand eine Auflösung von 2448 x 2048 Pixel. Die Intensitätswerte wären dann in dem ersten und dem zweiten Betriebszustand des zweiten Beispiels bei gleicher Intensität der Fluoreszenzstrahlung gleich.

Alternativ können in dem zweiten Beispiel in dem zweiten Betriebszustand temporäre Digitalbilder mit jeweiligen Teilerfassungszeitdauern von 19 ms gewonnen werden und das weitere Digitalbild durch Mittelung der temporären Digitalbilder gewonnen. Es wird dann eine Verstärkung der Sensorpixelwerte mit einem Linearfaktor von 300/19 vorgenommen. Jeweils neun Sensorpixelwerte werden dann mittels De-Bayering zur Bestimmung einer RGB-Information eines einzelnen Pixels zusammengefasst, ohne eine Auflösungsreduktion herbeizuführen. Das resultierende Bild hat dann in dem ersten Betriebszustand eine Auflösung von 2448 x 2048 Pixel. Die Intensitätswerte wären dann auch hier in dem ersten und dem zweiten Betriebszustand des zweiten Beispiels bei gleicher Intensität der Fluoreszenzstrahlung gleich.

Die Figuren 8 und 9 zeigen Beispielbilder von Immunfluoreszenzbildern von HEP-Zellen, welche ursprünglich mittels eines Farb-Bildsensors gewonnen wurden. Der verwendete Fluoreszenzfarbstoff war in diesem Fall Fluorescein Isothiocyanate (FITC). Die Fluoreszenzstrahlung wurde mittels eines CMOS-Sensors erfasst. Die Leistungsdichte der Anregungsstrahlung betrug 65 mW/mm². In dem ersten Betriebszustand betrug die Erfassungszeitdauer ca. 5,07 ms, die Verstärkung betrug ca. 32,5 dB und es wurde ein De-Bayering mit je 4 Pixeln und gleichzeitiger Auflösungsreduktion durchgeführt, so dass die Bildauflösung ¼ (ein Viertel) der Bildauflösung des zweiten Betriebszustandes entsprach. In dem zweiten Betriebszustand betrug die Erfassungszeitdauer ca. 162,12 ms, die Verstärkung betrug ca. 2,4 dB und es wurde ein De-Bayering mit je 9 Pixeln ohne Auflösungsreduktion durchgeführt, so dass die Bildauflösung der vierfachen Bildauflösung des ersten Betriebszustandes entsprach. Für beide Betriebszustände wurde bei gleicher Intensität der Fluoreszenzstrahlung eine gleiche Bildintensität erreicht.

Die Figuren 10 und 11 zeigen Beispielbilder von Immunfluoreszenzbildern von Gewebeteilen einer Rattenleber, welche ursprünglich mittels eines Farb-Bildsensors gewonnen wurden. In dem ersten Betriebszustand betrug die Erfassungszeitdauer ca. 8,93 ms, die Verstärkung betrug ca. 10,92 dB und es wurde ein De-Bayering mit je 4 Pixeln und gleichzeitiger Auflösungsreduktion durchgeführt, so dass die Bildauflösung ¼ (ein Viertel) der Bildauflösung des zweiten Betriebszustandes entsprach. In dem zweiten Betriebszustand betrug die Erfassungszeitdauer ca. 142,8 ms, die Verstärkung betrug ca. 35,00 dB und es wurde ein De-Bayering mit je 9 Pixeln ohne Auflösungsreduktion durchgeführt, so dass die Bildauflösung der vierfachen Bildauflösung des ersten Betriebszustandes entsprach. Für beide Betriebszustände wurde bei gleicher Intensität der Fluoreszenzstrahlung eine gleiche Bildintensität erreicht.

Die in der vorstehenden Beschreibung, den Ansprüchen und den Zeichnungen offenbarten Merkmale können sowohl einzeln als auch in beliebiger Kombination für die Verwirklichung von Ausführungsbeispielen in ihren verschiedenen Ausgestaltungen von Bedeutung sein und - soweit sich nicht aus der Beschreibung etwas anderes ergibt - beliebig miteinander kombiniert werden.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar.

Je nach bestimmten Implementierungsanforderungen können Ausführungsbeispiele der Erfindung in Hardware oder in Software implementiert sein, insbesondere die hier erwähnte Steuereinheit. Die Implementierung kann unter Verwendung eines digitalen Speichermediums, beispielsweise einer Floppy-Disk, einer DVD, einer Blu-Ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einer programmierbaren Hardwarekomponente derart zusammenwirken können oder zusammenwirken, dass das jeweilige Verfahren durchgeführt wird.

Eine programmierbare Hardwarekomponente wie die hier erwähnte Steuereinheit kann durch einen Prozessor, einen Computerprozessor (CPU = Central Processing Unit), einen Grafikprozessor (GPU = Graphics Processing Unit), einen Computer, ein Computersystem, einen anwendungsspezifischen integrierten Schaltkreis (ASIC = Application-Specific Integrated Circuit), einen integrierten Schaltkreis (IC = Integrated Circuit), ein Ein-Chip-System (SOC = System on Chip), ein programmierbares Logikelement oder ein feldprogrammierbares Gatterarray mit einem Mikro-prozessor (FPGA = Field Programmable Gate Array) gebildet sein.

Das digitale Speichermedium kann daher maschinen- oder computerlesbar sein. Manche Ausführungsbeispiele umfassen also einen Datenträger, der elektronisch lesbare Steuersignale aufweist, die in der Lage sind, mit einem programmierbaren Computersystem oder einer programmierbare Hardwarekomponente derart zusammenzuwirken, dass eines der hierin beschriebenen Verfahren durchgeführt wird. Ein Ausführungsbeispiel ist somit ein Datenträger (oder ein digitales Speichermedium oder ein computerlesbares Medium), auf dem das Programm zum Durchführen eines der hierin beschriebenen Verfahren aufgezeichnet ist.

Allgemein können Ausführungsbeispiele der vorliegenden Erfindung als Programm, Firmware, Computerprogramm oder Computerprogrammprodukt mit einem Programmcode oder als Daten implementiert sein, wobei der Programmcode oder die Daten dahin gehend wirksam ist bzw. sind, eines der Verfahren durchzuführen, wenn das Programm auf einem Prozessor oder einer programmierbaren Hardwarekomponente abläuft. Der Programmcode oder die Daten kann bzw. können beispielsweise auch auf einem maschinenlesbaren Träger oder Datenträger gespeichert sein. Der Programmcode oder die Daten können unter anderem als Quellcode, Maschinencode oder Bytecode sowie als anderer Zwischencode vorliegen.

Ein Programm gemäß eines Ausführungsbeispiels kann eines der Verfahren während seiner Durchführung beispielsweise dadurch umsetzen, dass dieses Speicherstellen ausliest oder in diese ein Datum oder mehrere Daten hinein schreibt, wodurch gegebenenfalls Schaltvorgänge oder andere Vorgänge in Transistorstrukturen, in Verstärkerstrukturen oder in anderen elektrischen, optischen, magnetischen oder nach einem anderen Funktionsprinzip arbeitenden Bauteile hervorgerufen werden. Entsprechend können durch ein Auslesen einer Speicherstelle Daten, Werte, Sensorwerte oder andere Informationen von einem Programm erfasst, bestimmt oder gemessen werden. Ein Programm kann daher durch ein Auslesen von einer oder mehreren Speicherstellen Größen, Werte, Messgrößen und andere Informationen erfassen, bestimmen oder messen, sowie durch Schreiben in eine oder mehrere Speicherstellen eine Aktion bewirken, veranlassen oder durchführen sowie andere Geräte, Maschinen und Komponenten ansteuern.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

## Patentansprüche

1. Verfahren zum Erfassen und Darstellen eines Immunfluoreszenzbildes (BI1, BI2, BI3) einer biologischen Probe (G),
aufweisend in einem ersten Betriebszustand (BZ1)
- fortdauerndes Beleuchten der Probe (G) mit einer Anregungsstrahlung (AS),
- Verändern einer Relativposition zwischen der Probe (G) und einem optischen System (OS), welches von der Probe (G) emittierte Fluoreszenzstrahlung (FS) hin zu wenigstens einem Bildsensor (BS) führt, in Abhängigkeit einer Bewegungsgeste (BG1) eines Nutzers,
- Erfassen der Fluoreszenzstrahlung (FS) mittels mehrerer Sensorpixel (P1, P2, P3, P4, P5, P6, P7, P8, P9, P10, P11, P12, P13, P14) des wenigstens einen Bildsensors (BS) unter Verwendung einer ersten Erfassungszeitdauer und Bestimmen eines Digitalbildes (BI1, BI2) sowie
- Anzeigen des Digitalbildes (BI1, BI2),
wobei in dem ersten Betriebszustand (BZ1) das Erfassen der Fluoreszenzstrahlung (FS) sowie das Bestimmen und das Anzeigen des Digitalbildes (BI1, BI2) zu aufeinander folgenden Zeitpunkten mit einer bestimmten Wiederholungsfrequenz wiederholt wird,
wobei ferner bei Detektieren einer Beendigung der Bewegungsgeste (BG1) von dem ersten Betriebszustand (BZ1) in einen zweiten Betriebszustand (BZ2) gewechselt wird,
ferner aufweisend in dem zweiten Betriebszustand (BZ2)
- Beleuchten der Probe (G) mit der Anregungsstrahlung (AS),
- Erfassen von von der Probe (G) emittierter Fluoreszenzstrahlung (FS) mittels der mehreren Sensorpixel (P1, P2, P3, P4, P5, P6, P7, P8, P9, P10, P11, P12, P13, P14) unter Verwendung einer zweiten Erfassungszeitdauer und Bestimmen eines weiteren Digitalbildes (BI3),
- Anzeigen des weiteren Digitalbildes (BI3),
- Beenden des Erfassens der emittierten Fluoreszenzstrahlung (FS) und Beenden des Beleuchtens der Probe (G) nach Ablauf der zweiten Erfassungszeitdauer
- fortdauerndes Anzeigen des weiteren Digitalbildes (BI3) nach dem Beenden des Erfassens der Fluoreszenzstrahlung (FS) und dem Beenden des Beleuchtens der Probe (G),
**dadurch gekennzeichnet, dass** die zweite Erfassungszeitdauer größer ist als die erste Erfassungszeitdauer.

2. Verfahren nach Anspruch 1,
ferner aufweisend
- Beenden des zweiten Betriebszustandes (BZ2) und Übergehen in den ersten Betriebszustand (BZ1) bei Detektieren eines Beginnens einer neuen Bewegungsgeste (BG2) des Nutzers.

3. Verfahren nach Anspruch 1,
wobei das Digitalbild (BI1, BI2) in dem ersten Betriebszustand (BZ1) und das weitere Digitalbild (BI3) in dem zweiten Betriebszustand (BZ2) so bestimmt werden, dass bei gleicher Lichtintensität der Fluoreszenzstrahlung (FS) das Digitalbild (BI1, BI2) des ersten Betriebszustandes (BZ1) und das weitere Digitalbild (BI3) des zweiten Betriebszustand (BZ2) eine gleiche Intensität aufweisen.

4. Verfahren nach Anspruch 3,
wobei einer oder mehrere der folgenden Parameter für den ersten Betriebszustand (BZ1) und für den zweiten Betriebszustand (BZ2) unterschiedlich gewählt wird:
- Verstärkungsfaktor für Sensorpixelwerte (SW),
- Anzahl von Sensorpixelwerten (SW), welche mittels Binning zu einem Bildpixelwert zusammengefasst werden,
- Anzahl von Sensorpixelwerten (SW), welche mittels Debayering zu einem Bildpixelwert zusammengefasst werden.

5. Verfahren nach Anspruch 1,
wobei das Digitalbild (BI1, BI2) in dem ersten Betriebszustand (BZ1) so bestimmt wird, dass es eine erste Bildauflösung aufweist,
wobei das weitere Digitalbild (BI3) in dem zweiten Betriebszustand (BZ2) so bestimmt wird, dass es eine zweite Bildauflösung aufweist,
und wobei die erste Bildauflösung geringer als die zweite Bildauflösung gewählt wird.

6. Verfahren nach Anspruch 1,
wobei in dem zweiten Betriebszustand (BZ2) das Erfassen der emittierten Fluoreszenzstrahlung (FS) unter Verwendung mehrerer, aufeinander folgender Teil-Erfassungszeitdauern innerhalb der zweiten Erfassungszeitdauer erfolgt,
wobei für die Teil-Erfassungszeitdauern korrespondierende, temporäre Digitalbilder bestimmt werden
und wobei das weitere Digitalbild (BI3) auf Basis der temporären Digitalbilder bestimmt wird.

7. Verfahren nach Anspruch 6,
wobei in dem zweiten Betriebszustand (BZ2) bei Detektieren eines Beginnens einer neuen Bewegungsgeste (BG2) des Nutzers das Erfassen der emittierten Fluoreszenzstrahlung (FS) und das Bestimmen der temporären Digitalbilder abgebrochen wird und in den ersten Betriebszustand (BZ2) übergegangen wird.

8. Verfahren nach Anspruch 1,
wobei als Bildsensor (BS) ein Farbbildsensor (BS1) verwendet wird.

9. Verfahren nach Anspruch 1,
wobei als Bildsensor (BS) ein Grauwert-Bildsensor (BS2) verwendet wird, welcher Fluoreszenzstrahlung (FS2) in einem Grünkanal detektiert.

10. Verfahren nach Anspruch 9,
wobei ferner ein weiterer Grauwert-Bildsensor (BS3) verwendet wird, welcher Fluoreszenzstrahlung (FS3) in einem Rotkanal detektiert.

11. Verfahren nach Anspruch 1,
wobei bei einem Wechseln von dem ersten Betriebszustand (BZ1) in den zweiten Betriebszustand (BZ2) vor Einnehmen des zweiten Betriebszustandes (BZ2) ein Fokussieren des optischen Systems (OS) auf die Probe (G) durchgeführt wird.

12. Vorrichtung (V) zum Erfassen und Darstellen eines Immunfluoreszenzbildes (BI1, BI2, BI3) einer biologischen Probe (G),
aufweisend
- eine Anregungslichtquelle (AL) zum Beleuchten der Probe (G) mit einer Anregungsstrahlung (AS),
- eine Haltevorrichtung (H) zum Halten der Probe (G),
- wenigstens einen Bildsensor (BS) mit mehreren Sensorpixeln (P1, P2, P3, P4, P5, P6, P7, P8, P9, P10, P11, P12, P13, P14) zum Erfassen von von der Probe (G) emittierter Fluoreszenzstrahlung (FS),
- ein optisches System (OS) zum Führen der Fluoreszenzstrahlung (FS) von der Probe (G) hin zu dem Bildsensor (BS),
- einer Positioniereinheit (P), welche ausgebildet ist, eine Relativposition zwischen der Probe (G) und einem optischen System (OS) zu verändern,
- wenigstens eine Steuereinheit (S) mit einer ersten Schnittstelle (SC1) zu einer Nutzereingabevorrichtung (N), einer zweiten Schnittstelle (SC2) zu dem Bildsensor (BS), einer dritten Schnittstelle (SC3) zu der Anregungslichtquelle (AL), einer vierten Schnittstelle (SC4) zu der Positioniereinheit (P) und einer fünften Schnittstelle (SC5) zu einer Anzeigeeinheit (AE),
wobei die Steuereinheit (S) ausgebildet ist, in einem ersten Betriebszustand (BZ1)
- die Anregungslichtquelle (AL) derart anzusteuern, dass die Probe (G) fortdauernd mit der Anregungsstrahlung (AS) beleuchtet wird,
- ferner aus einem Eingabesignal (ES) der Nutzereingabevorrichtung (N) eine Bewegungsgeste (BG1) des Nutzers abzuleiten und die Positioniereinheit (P) so anzusteuern, dass die Relativposition zwischen der Probe (G) und dem optischen System (OS) in Abhängigkeit der Bewegungsgeste (BG1) verändert wird,
- den Bildsensor (BS) so anzusteuern, dass mittels der mehreren Sensorpixel (P1, P2, P3, P4, P5, P6, P7, P8, P9, P10, P11, P12, P13, P14) die Fluoreszenzstrahlung (FS) unter Verwendung einer ersten Erfassungszeitdauer erfasst wird und ferner aus resultierenden Sensorpixelwerten (SW) ein Digitalbild (BI1, BI2) zu bestimmen sowie ferner
- die Anzeigeeinheit (AE) zum Anzeigen des Digitalbildes (BI1, BI2) anzusteuern,
wobei die Steuereinheit (S) ferner ausgebildet ist, in dem ersten Betriebszustand (BZ1)
- das Erfassen der Fluoreszenzstrahlung (FS) sowie das Bestimmen und das Anzeigen des Digitalbildes (BI1, BI2) zu aufeinander folgenden Zeitpunkten mit einer bestimmten Wiederholungsfrequenz zu wiederholen
- und bei Detektieren einer Beendigung der Bewegungsgeste (BG1) in einen zweiten Betriebszustand (BZ2) zu wechseln,
wobei die Steuereinheit (S) ferner ausgebildet ist, in dem zweiten Betriebszustand (BZ2)
- den Bildsensor (BS) so anzusteuern, dass mittels der mehreren Sensorpixel (P1, P2, P3, P4, P5, P6, P7, P8, P9, P10, P11, P12, P13, P14) die Fluoreszenzstrahlung (FS) unter Verwendung einer zweiten Erfassungszeitdauer erfasst wird und ferner aus resultierenden Sensorpixelwerten (SW) ein weiteres Digitalbild (BI3) zu bestimmen, die Anzeigeeinheit (AE) zum Anzeigen des weiteren Digitalbildes (BI3) anzusteuern,
- ferner den Bildsensor (BS) so anzusteuern, dass nach Ablauf der zweiten Erfassungszeitdauer das Erfassen der Fluoreszenzstrahlung (FS) unterlassen wird sowie ferner die Anregungslichtquelle (AL) derart anzusteuern, dass das Beleuchten der Probe (G) nach Ablauf der zweiten Erfassungszeitdauer beendet wird
- und schließlich ferner die Anzeigeeinheit (AE) nach dem Beenden des Beleuchtens der Probe (G) und nach dem Beenden des Erfassens der emittierten Fluoreszenzstrahlung (FS) derart anzusteuern, dass das weitere Digitalbild (BI3) fortdauernd angezeigt wird.
**dadurch gekennzeichnet, dass** die zweite Erfassungszeitdauer größer ist als die erste Erfassungszeitdauer.

13. Vorrichtung nach Anspruch 12,
wobei die Steuereinheit (S) ferner ausgebildet ist, bei Detektieren eines Beginnens einer neuen Bewegungsgeste (BG2) des Nutzers den zweiten Betriebszustand (BZ2) zu beenden und in den ersten Betriebszustand (BZ1) überzugehen.

## Claims

1. Method for acquiring and displaying an immunofluorescence image (BI1, BI2, BI3) of a biological sample (G),
comprising in a first operating state (BZ1)
- continuously illuminating the sample (G) using excitation radiation (AS),
- changing a relative position between the sample (G) and an optical system (OS), which guides fluorescent radiation (FS) emitted by the sample (G) onto at least one image sensor (BS), in dependence on a movement gesture (BG1) of a user,
- acquiring the fluorescent radiation (FS) by means of multiple sensor pixels (P1, P2, P3, P4, P5, P6, P7, P8, P9, P10, P11, P12, P13, P14) of the at least one image sensor (BS) using a first acquisition duration and determining a digital image (BI1, BI2), and
- displaying the digital image (BI1, BI2),
wherein in the first operating state (BZ1), the acquisition of the fluorescent radiation (FS) as well as the determination and the display of the digital image (BI1, BI2) are repeated at successive points in time at a defined repetition frequency,
wherein furthermore upon detection of a termination of the movement gesture (BG1), a change is made from the first operating state (BZ1) to a second operating state (BZ2),
furthermore comprising in the second operating state (BZ2)
- illuminating the sample (G) using the excitation radiation (AS),
- acquiring fluorescent radiation (FS) emitted by the sample (G) by means of the multiple sensor pixels (P1, P2, P3, P4, P5, P6, P7, P8, P9, P10, P11, P12, P13, P14) using a second acquisition duration and determining a further digital image (BI3),
- displaying the further digital image (BI3),
- terminating the acquisition of the emitted fluorescent radiation (FS) and terminating the illumination of the sample (G) after lapse of the second acquisition duration,
- continuously displaying the further digital image (BI3) after the termination of the acquisition of the fluorescent radiation (FS) and the termination of the illumination of the sample (G),
**characterized in that** the second acquisition duration is greater than the first acquisition duration.

2. Method according to Claim 1,
furthermore comprising
- terminating the second operating state (BZ2) and changing to the first operating state (BZ1) upon detection of a beginning of a new movement gesture (BG2) of the user.

3. Method according to Claim 1,
wherein the digital image (BI1, BI2) in the first operating state (BZ1) and the further digital image (BI3) in the second operating state (BZ2) are determined such that at equal light intensity of the fluorescent radiation (FS), the digital image (BI1, BI2) of the first operating state (BZ1) and the further digital image (BI3) of the second operating state (BZ2) have an equal intensity.

4. Method according to Claim 3,
wherein one or more of the following parameters being chosen differently for the first operating state (BZ1) and for the second operating state (BZ2):
- amplification factor for sensor pixel values (SW),
- number of sensor pixel values (SW) which are combined by means of binning to form one image pixel value,
- number of sensor pixel values (SW) which are combined by means of debayering to form one image pixel value.

5. Method according to Claim 1,
wherein the digital image (BI1, BI2) is determined in the first operating state (BZ1) such that it has a first image resolution,
wherein the further digital image (BI3) is determined in the second operating state (BZ2) such that it has a second image resolution,
and wherein the first image resolution is chosen smaller than the second image resolution.

6. Method according to Claim 1,
wherein in the second operating state (BZ2), the acquisition of the emitted fluorescent radiation (FS) is carried out using multiple successive partial acquisition durations within the second acquisition duration,
wherein corresponding temporary digital images are determined for the partial acquisition durations,
and wherein the further digital image (BI3) is determined on the basis of the temporary digital images.

7. Method according to Claim 6,
wherein in the second operating state (BZ2), upon detection of a beginning of a new movement gesture (BG2) of the user, the acquisition of the emitted fluorescent radiation (FS) and the determination of the temporary digital images is terminated and a transition is made into the first operating state (BZ2).

8. Method according to Claim 1,
wherein a colour image sensor (BS1) is used as the image sensor (BS).

9. Method according to Claim 1,
wherein a greyscale image sensor (BS2), which detects fluorescent radiation (FS2) in a green channel, is used as the image sensor (BS).

10. Method according to Claim 9,
wherein furthermore a further greyscale image sensor (BS3) is used, which detects fluorescent radiation (FS3) in a red channel.

11. Method according to Claim 1,
wherein upon a change from the first operating state (BZ1) to the second operating state (BZ2), before reaching the second operating state (BZ2), a focusing of the optical system (OS) on the sample (G) is carried out.

12. Device (V) for acquiring and displaying an immunofluorescence image (BI1, BI2, BI3) of a biological sample (G),
comprising
- an excitation light source (AL) for illuminating the sample (G) using excitation radiation (AS),
- a holding device (H) for holding the sample (G),
- at least one image sensor (BS) having multiple sensor pixels (P1, P2, P3, P4, P5, P6, P7, P8, P9, P10, P11, P12, P13, P14) for acquiring fluorescent radiation (FS) emitted by the sample (G),
- an optical system (OS) for guiding the fluorescent radiation (FS) from the sample (G) onto the image sensor (BS),
- a positioning unit (P), which is designed to change a relative position between the sample (G) and an optical system (OS),
- at least one control unit (S) having a first interface (SC1) to a user input device (N), a second interface (SC2) to the image sensor (BS), a third interface (SC3) to the excitation light source (AL), a fourth interface (SC4) to the positioning unit (P), and a fifth interface (SC5) to a display unit (AE),
wherein the control unit (S) is designed, in a first operating state (BZ1)
- to control the excitation light source (AL) in such a way that the sample (G) is continuously illuminated using the excitation radiation (AS),
- furthermore to derive a movement gesture (BG1) of the user from an input signal (ES) of the user input device (N) and to control the positioning unit (P) in such a way that the relative position between the sample (G) and the optical system (OS) is changed in dependence on the movement gesture (BG1),
- to control the image sensor (BS) in such a way that, by means of the multiple sensor pixels (P1, P2, P3, P4, P5, P6, P7, P8, P9, P10, P11, P12, P13, P14), the fluorescent radiation (FS) is acquired using a first acquisition duration and furthermore to determine a digital image (BI1, BI2) from resulting sensor pixel values (SW) and also furthermore
- to control the display unit (AE) to display the digital image (BI1, BI2),
wherein the control unit (S) is furthermore designed, in the first operating state (BZ1)
- to repeat the acquisition of the fluorescent radiation (FS) as well as the determination and the display of the digital image (BI1, BI2) at successive points in time using a defined repetition frequency
- and upon detection of a termination of the movement gesture (BG1), to change to a second operating state (BZ2),
wherein the control unit (S) is furthermore designed, in the second operating state (BZ2)
- to control the image sensor (BS) so that by means of the multiple sensor pixels (P1, P2, P3, P4, P5, P6, P7, P8, P9, P10, P11, P12, P13, P14), the fluorescent radiation (FS) is acquired using a second acquisition duration and furthermore to determine a further digital image (BI3) from resulting sensor pixel values (SW), and to control the display unit (AE) to display the further digital image (BI3),
- and furthermore to control the image sensor (BS) so that after lapse of the second acquisition duration, the acquisition of the fluorescent radiation (FS) is terminated, and also furthermore to control the excitation light source (AL) in such a way that the illumination of the sample (G) is terminated after lapse of the second acquisition duration,
- and finally furthermore to control the display unit (AE) after the termination of the illumination of the sample (G) and after the termination of the acquisition of the emitted fluorescent radiation (FS) in such a way that the further digital image (BI3) is continuously displayed
**characterized in that** the second acquisition duration is greater than the first acquisition duration.

13. Device according to Claim 12,
wherein the control unit (S) is furthermore designed, upon detection of a beginning of a new movement gesture (BG2) of the user, to end the second operating state (BZ2) and to changeto the first operating state (BZ1).

## Revendications

1. Procédé pour acquérir et représenter une image d'immunofluorescence (BI1, BI2, BI3) d'un échantillon biologique (G),
comprenant, dans un premier état opérationnel (BZ1),
- éclairage continu de l'échantillon (G) avec un rayonnement d'excitation (AS),
- modification de la position relative entre l'échantillon (G) et un système optique (OS) qui guide le rayonnement de fluorescence (FS) émis depuis l'échantillon (G) vers au moins un capteur d'image (BS), en fonction d'un geste de déplacement (BG1) d'un utilisateur,
- acquisition du rayonnement de fluorescence (FS) au moyen de plusieurs pixels de capteur (P1, P2, P3, P4, P5, P6, P7, P8, P9, P10, P11, P12, P13, P14) de l'au moins un capteur d'image (BS) en utilisant une première durée d'acquisition et détermination d'une image numérique (BI1, BI2) et aussi
- affichage de l'image numérique (BI1, BI2),
dans le premier état opérationnel (BZ1), l'acquisition du rayonnement de fluorescence (FS) ainsi que la détermination et l'affichage de l'image numérique (BI1, BI2) étant répétés à des instants successifs avec une fréquence de répétition déterminée,
en outre, lors de la détection d'une fin du geste de déplacement (BG1), un basculement étant effectué du premier état opérationnel (BZ1) en un deuxième état opérationnel (BZ2),
comprenant en outre, dans le deuxième état opérationnel (BZ2),
- éclairage de l'échantillon (G) avec le rayonnement d'excitation (AS),
- acquisition du rayonnement de fluorescence (FS) émis par l'échantillon (G) au moyen de plusieurs pixels de capteur (P1, P2, P3, P4, P5, P6, P7, P8, P9, P10, P11, P12, P13, P14) en utilisant une deuxième durée d'acquisition et détermination d'une image numérique supplémentaire (BI3),
- affichage de l'image numérique supplémentaire (BI3),
- achèvement de l'acquisition du rayonnement de fluorescence (FS) émis et achèvement de l'éclairage de l'échantillon (G) après écoulement de la deuxième durée d'acquisition,
- affichage continu de l'image numérique supplémentaire (BI3) après l'achèvement de l'acquisition du rayonnement de fluorescence (FS) émis et l'achèvement de l'éclairage de l'échantillon (G),
**caractérisé en ce que** la deuxième durée d'acquisition est supérieure à la première durée d'acquisition.

2. Procédé selon la revendication 1,
comprenant en outre
- achèvement du deuxième état opérationnel (BZ2) et basculement dans le premier état opérationnel (BZ1) lors de la détection d'un début d'un nouveau geste de déplacement (BG2) de l'utilisateur.

3. Procédé selon la revendication 1,
l'image numérique (BI1, BI2) dans le premier état opérationnel (BZ1) et l'image numérique supplémentaire (BI3) dans le deuxième état opérationnel (BZ2) étant déterminées de telle sorte qu'avec une intensité lumineuse identique du rayonnement de fluorescence (FS), l'image numérique (BI1, BI2) dans le premier état opérationnel (BZ1) et l'image numérique supplémentaire (BI3) dans le deuxième état opérationnel (BZ2) présentent une même intensité.

4. Procédé selon la revendication 3,
un ou plusieurs des paramètres suivants étant sélectionnés différemment pour le premier état opérationnel (BZ1) et le deuxième état opérationnel (BZ2):
- facteur de gain pour les valeurs de pixel de capteur (SW),
- nombre de valeurs de pixel de capteur (SW) qui sont regroupées en une valeur de pixel d'image au moyen d'un groupement de données par classe,
- nombre de valeurs de pixel de capteur (SW) qui sont regroupées en une valeur de pixel d'image au moyen du dématriçage.

5. Procédé selon la revendication 1,
l'image numérique (BI1, BI2) dans le premier état opérationnel (BZ1) étant déterminée de telle sorte qu'elle présente une première résolution d'image, l'image numérique supplémentaire (BI3) dans le deuxième état opérationnel (BZ2) étant déterminée de telle sorte qu'elle présente une deuxième résolution d'image, et la première résolution d'image étant inférieure à la deuxième résolution d'image.

6. Procédé selon la revendication 1,
dans le deuxième état opérationnel (BZ2), l'acquisition du rayonnement de fluorescence (FS) émis étant effectué en utilisant plusieurs durées d'acquisition partielles successives au sein de la deuxième durée d'acquisition,
des images numériques temporaires correspondant aux durées d'acquisition partielles étant déterminées
et l'image numérique supplémentaire (BI3) étant déterminée sur la base des images numériques temporaires.

7. Procédé selon la revendication 6,
dans le deuxième état opérationnel (BZ2), lors de la détection d'un début d'un nouveau geste de déplacement (BG2) de l'utilisateur, l'acquisition du rayonnement de fluorescence (FS) émis et la détermination des images numériques temporaires étant interrompues et un basculement dans le premier état opérationnel (BZ2) ayant lieu.

8. Procédé selon la revendication 1,
le capteur d'image (BS) utilisé étant un capteur d'image couleur (BS1).

9. Procédé selon la revendication 1,
le capteur d'image (BS) utilisé étant un capteur d'image à niveaux de gris (BS2) qui détecte le rayonnement de fluorescence (FS) dans un canal vert.

10. Procédé selon la revendication 9,
un capteur d'image à niveaux de gris supplémentaire (BS3) étant utilisé, lequel détecte le rayonnement de fluorescence (FS) dans un canal rouge.

11. Procédé selon la revendication 1,
lors d'un changement du premier état opérationnel (BZ1) dans le deuxième état opérationnel (BZ2), une mise au point du système optique (OS) sur l'échantillon (G) étant effectuée avant l'adoption du deuxième état opérationnel (BZ2).

12. Dispositif (V) pour acquérir et représenter une image d'immunofluorescence (BI1, BI2, BI3) d'un échantillon biologique (G),
comprenant
- une source de lumière d'excitation (AL) destinée à éclairer l'échantillon (G) avec un rayonnement d'excitation (AS),
- un dispositif de maintien (H) destiné à maintenir l'échantillon (G),
- au moins un capteur d'image (BS) pourvu de plusieurs pixels de capteur (P1, P2, P3, P4, P5, P6, P7, P8, P9, P10, P11, P12, P13, P14) destiné à acquérir le rayonnement de fluorescence (FS) émis depuis l'échantillon (G),
- un système optique (OS) destiné à guider le rayonnement de fluorescence (FS) de l'échantillon (G) vers le capteur d'image (BS),
- une unité de positionnement (P), qui est configurée pour modifier une position relative entre l'échantillon (G) et un système optique (OS),
- au moins une unité de commande (S) pourvue d'une première interface (SC1) avec un dispositif de saisie d'utilisateur (N), une deuxième interface (SC2) avec le capteur d'image (BS), une troisième interface (SC3) avec la source de lumière d'excitation (AL), une quatrième interface (SC4) avec l'unité de positionnement (P) et une cinquième interface (SC5) avec une unité d'affichage (AE),
l'unité de commande (S) étant configurée pour, dans un premier état opérationnel (BZ1),
- commander la source de lumière d'excitation (AL) de telle sorte que l'échantillon (G) est continuellement éclairé avec le rayonnement d'excitation (AS),
- en outre, à partir d'un signal de saisie (ES) du dispositif de saisie d'utilisateur (N), dériver un geste de déplacement (BG1) de l'utilisateur et commander l'unité de positionnement (P) de telle sorte que la position relative entre l'échantillon (G) et le système optique (OS) est modifiée en fonction du geste de déplacement (BG1),
- commander le capteur d'image (BS) de telle sorte que le rayonnement de fluorescence (FS) soit acquis au moyen des plusieurs pixels de capteur (P1, P2, P3, P4, P5, P6, P7, P8, P9, P10, P11, P12, P13, P14) en utilisant une première durée d'acquisition et, en outre, en vue de déterminer une image numérique (BI1, BI2) à partir des valeurs de pixel de capteur (SW) résultantes, et en outre
- commander l'unité d'affichage (AE) pour afficher l'image numérique (BI1, BI2),
l'unité de commande (S) étant en outre configurée pour, dans le premier état opérationnel (BZ1),
- répéter l'acquisition du rayonnement de fluorescence (FS) ainsi que la détermination et l'affichage de l'image numérique (BI1, BI2) à des instants successifs avec une fréquence de répétition déterminée,
- et, lors de la détection d'une fin du geste de déplacement (BG1), basculer dans un deuxième état opérationnel (BZ2),
l'unité de commande (S) étant en outre configurée pour, dans le deuxième état opérationnel (BZ2),
- commander le capteur d'image (BS) de telle sorte que le rayonnement de fluorescence (FS) soit acquis moyen des plusieurs pixels de capteur (P1, P2, P3, P4, P5, P6, P7, P8, P9, P10, P11, P12, P13, P14) en utilisant une deuxième durée d'acquisition et, en outre, en vue de déterminer une image numérique supplémentaire (BI3) à partir des valeurs de pixel de capteur (SW) résultantes, commander l'unité d'affichage (AE) pour afficher l'image numérique supplémentaire (BI3),
- commander en outre le capteur d'image (BS) de telle sorte qu'après l'écoulement de la deuxième durée d'acquisition, l'acquisition du rayonnement de fluorescence (FS) soit abandonnée et, en outre, commander la source de lumière d'excitation (AL) de telle sorte que l'éclairage de l'échantillon (G) prenne fin après l'écoulement de la deuxième durée d'acquisition
- et finalement commander en outre l'unité d'affichage (AE) après la fin de l'éclairage de l'échantillon (G) et après la fin de l'acquisition du rayonnement de fluorescence (FS) émis de telle sorte que l'image numérique supplémentaire (BI3) soit affichée en continu,
**caractérisé en ce que** la deuxième durée d'acquisition est supérieure à la première durée d'acquisition.

13. Dispositif selon la revendication 12,
l'unité de commande (S) étant en outre configurée pour, lors de la détection d'un début d'un nouveau geste de déplacement (BG2) de l'utilisateur, mettre fin au deuxième état opérationnel (BZ2) et basculer dans le premier état opérationnel (BZ1).
